# EUROPEAN PATENT APPLICATION

(11) **EP 4 124 347 A1**
(43) Date of publication of application: **01.02.2023**
(21) Application number: 22187715.2
(22) Date of filing: 29.07.2022
(51) Int. Cl.: A61K 48/00, C12N 15/11

(54) **CIRCULAR RNA MOLECULE AND USE THEREOF IN TARGETED DEGRADATION OF PROTEIN OF INTEREST**

(30) Priority: 29.07.2021 CN 202110863519
(71) Applicant: Suzhou Curemed Biomedical Technology Co. Ltd, Suzhou, Jiangsu 215123 (CN)
(72) Inventor: ZUO, Chijian, Suzhou, 215123 (CN); YANG, Jiali, Suzhou, 215123 (CN); ZHU, Jiafeng, Suzhou, 215123 (CN); DU, Yaran, Suzhou, 215123 (CN); ZHAO, Yang, Suzhou, 215123 (CN); SUN, Zhenhua, Suzhou, 215123 (CN)
(74) Representative: Heubeck, Christian

(57) **Abstract**

The present disclosure belongs to the field of biomedicine. Specifically, the present disclosure relates to a circular RNA molecule, a cyclization precursor RNA molecule, a recombinant nucleic acid molecule, a recombinant expression vector, a recombinant host cell, a composition and use thereof in targeted degradation of a protein of interest, as well as a method for preventing or treating a disease. The circular RNA molecule has good membrane permeability, is easily delivered into cells, and has high-efficiency *in vivo* protein targeted degradation activity. The circular RNA molecule of the present disclosure successfully achieves inhibition of tumor growth, which proves the *in vivo* protein degradation activity of bio-PROTACs for the first time, and provides a positive and effective treatment solution for the treatment of diseases such as tumor.

## Description

### CROSS-REFERENCE TO RELATED APPLICATIONS

The present application claims priority to the Chinese Patent Application No. 202110863519.2 filled on July 29, 2021, which is incorporated herein by reference in its entirety.

### TECHNICAL FIELD

The present disclosure belongs to the field of biomedicine. Specifically, the present disclosure relates to a circular RNA molecule, a cyclization precursor RNA molecule, a recombinant nucleic acid molecule, a recombinant expression vector, a recombinant host cell, a composition and use thereof in targeted degradation of a protein of interest, as well as a method for preventing or treating a disease.

### BACKGROUND

A protein in a cell is constantly in a process of metabolic turnover of synthesis, modification and degradation. Maintaining normal protein metabolism is essential for a normal function of life. Degradation or intervention of a specific protein is an important way to study the function of the protein, and is also an important measure to treat a related disease caused by abnormal protein expression. Currently, the degradation or intervention of a pathogenic protein has become one of the most effective disease treatment strategies.

A traditional small molecule inhibitor can affect the biological function of the pathogenic protein by binding to an active site of a protein of interest (POI), so as to achieve a therapeutic effect. So far, it has been successfully used in clinical treatment of various disease such as cancer and the like. However, the research and development strategy of traditional small molecule drugs determines that they cannot act on "undruggable" proteins of interest, resulting in that currently only about 20% of proteins in cells can be used as targets to be applied in clinical research of small molecule drugs. Meanwhile, a gene editing technology (e.g., CRISPR/Cas technology) and a RNAi technology both have down-regulated the expression of the protein of interest to some extent. However, the disadvantages of these methods are that it takes a long time to effectively eliminate protein expression, and they cannot act on post-translational modified proteins. Once the translation of the protein of interest stops, the consumption of protein depends entirely on its inherent half-life. Therefore, the methods such as CRISPR/Cas technology, the RNAi technology and the like have great application limitations in regulating the protein of interest^{[1]}.

Homeostasis of the proteins in the cells is maintained mainly depending on the coordinated operation of intrinsic protein degradation pathways in the cells, wherein an autophagy-lysosome pathway and an ubiquitin-proteasome pathway (UPP) play important biological functions in protein degradation.

A proteolysis-targeting chimera (PROTAC) technology is an effective tool for endogenous protein degradation developed in recent years^{[2-3]}. It mainly consists of three parts: 1) a E3 ligase ligand, which is mainly used for recruiting a E3 ligase; 2) a ligand of a protein of interest (POI), which can bind to the POI; and 3) a linker, which connects the E3 ligase ligand and the ligand of the POI. When the PROTAC enters cells, the ligand of the POI at one end thereof specifically recognizes and binds to the protein of interest, and the E3 ligase ligand at the other end thereof binds to E3, thereby forming a POI-PROTAC-E3 ligase ternary complex. The PROTAC complex can connect the E3 ligase onto the protein of interest, and recruit ubiquitinated E2, finally degrading the protein of interest by ubiquitination. Traditional PROTAC protein degradation agents are mainly small molecular compounds. The appearance of small molecule PROTACs protein degradation agents provides the possibility to solve an undruggable target, and thus they have also become a hot field of drug research and development. However, the drug research and development of small molecule PROTACs also has obvious shortcomings, for example their molecular weights are larger than those of traditional small molecule drugs, so they are difficult to penetrate a cell membrane and enter cells to play a role; and the small molecule PROTACs rely on the existing E3 ubiquitin ligases in the cells, and if the expression level of the latter is relatively low, it is difficult to produce an expected effect.

An antibody- or polypeptide-mediated protein degradation technology developed in recent years, referred to as bio-proteolysis targeting chimera (Bio-PROTAC) technology, can overcome the shortcomings of the aforementioned small molecule PROTACs. TRIM21 is a E3 ubiquitin ligase with high affinity to a Fc domain of an antibody^{[4]}, and is widely expressed in many cell types and tissues^{[5]}. TRIM21 can identify an antibody-bound pathogen and recruit a ubiquitin-proteasome system to degrade it^{[6]}. By utilizing the characteristics of TRIM21, a technology of degrading an endogenous protein, as generated by combining an antibody with the PROTAC technology, is called Trim-Away^{[7]}. In the Trim-Away system, the antibody is equivalent to a triplet structure of the PROTAC technology. After entering the cell through electrotransformation and the like, the antibody can bind to a specific protein of interest, and then the Fc terminal binds to TRIM21, such that the ternary complex of the antibody, the protein of interest and TRIM21 is ubiquitinated and finally degraded by a protease. There are three steps required to complete protein degradation: 1) introducing an antibody that can bind to a protein of interest; 2) recruiting endogenous or exogenous TRIM21 to a protein of interest; and 3) recruiting ubiquitinated E2 to promote proteasome-mediated degradation of a protein complex. This method can quickly degrade the protein of interest in cells, thereby avoiding a compensation pathway formed due to long-time screening. Compared with a traditional DNA and RNA level gene interference technology, the Trim-Away technology can achieve a better degradation effect on already produced proteins, especially long-lived proteins; and additionally, the Trim-Away technology can degrade both proteins in cytoplasm and nucleus. Furthermore, it has also been reported that the degradation of the protein of interest can be achieved by constructing a RING domain chimeric protein of nanoantibody-RNF4 E3 ubiquitin ligase^{[8]}; and others target the protein of interest by means of a single-chain antibody, Darpins, a nanoantibody, a polypeptide, etc., respectively and form chimeric proteins with different E3 ligases respectively to play a role in protein degradation. However, both Trim-Away and other various recombinant protein forms of Bio-PROTAC are faced with a major clinical application problem, that is, a protein degradation agent in the form of recombinant protein needs to be delivered into specific cells to play a role. The Bio-PROTAC has a high molecular weight and poor membrane permeability, which makes it difficult for the Bio-PROTAC to enter cells and play its role. For a cell line cultured *in vitro,* intracellular delivery of the recombinant protein can still be achieved by electrotransformation. However, there has been no suitable technical means for the *in vivo* delivery of the Bio-PROTAC molecule. Therefore, the *in vivo* application examples of the Bio-PROTAC have not been reported yet.

### References:

[1] Toyama, B.H., Savas, J.N., Park, S.K., Harris, M.S., Ingolia, N.T., Yates, J.R.,3rd, and Hetzer, M.W. (2013). Identification of long-lived proteins reveals exceptional stability of essential cellular structures. Cell 154, 971-982.
[2] Sakamoto KM, Kim KB, Verma R, Ransick A, Stein B, Crews CM, Deshaies RJ. Development of Protacs to target cancer-promoting proteins for ubiquitination and degradation. Mol Cell Proteomics. 2003 Dec;2(12): 1350-8.
[3] Burslem GM, Crews CM. Proteolysis-Targeting Chimeras as Therapeutics and Tools for Biological Discovery. Cell. 2020 Apr 2;181(1): 102-114.
[4] James LC, Keeble AH, Khan Z, Rhodes DA, Trowsdale J. Structural basis for PRYSPRY-mediated tripartite motif (TRIM) protein function. Proc Natl Acad Sci U S A. 2007 Apr 10;104(15):6200-5.
[5] Yoshimi R, Chang TH, Wang H, Atsumi T, Morse HC 3rd, Ozato K. Gene disruption study reveals a nonredundant role for TRIM21/Ro52 in NF-kappaB-dependent cytokine expression in fibroblasts. J Immunol. 2009 Jun 15;182(12):7527-38.
[6] Mallery DL, McEwan WA, Bidgood SR, Towers GJ, Johnson CM, James LC. Antibodies mediate intracellular immunity through tripartite motif-containing 21 (TRIM21). Proc Natl Acad Sci U S A. 2010 Nov 16;107(46):19985-90.
[7] Clift D, McEwan WA, Labzin LI, Konieczny V, Mogessie B, James LC, Schuh M. A Method for the Acute and Rapid Degradation of Endogenous Proteins. Cell. 2017 Dec 14;171(7):1692-1706.el8.
[8] Juríková M, Danihel L', Polák Š, Varga I. Ki67, PCNA, and MCM proteins: Markers of proliferation in the diagnosis of breast cancer. Acta Histochem. 2016 Jun;118(5):544-52.

### SUMMARY

### Problems to be solved by the present invention

In view of the problems existing in the prior art, for example, the recombinant protein of Bio-PROTAC has the defects of a large molecular weight, poor ability to enter cells, and inability to effectively deliver into the body and play an in-vivo protein degradation role. Therefore, the present disclosure provides a circular RNA molecule, which realizes the targeted degradation of a protein of interest for the first time, and provides a novel functional molecule for a technology of targeted degradation of proteins. The circular RNA molecule has good membrane permeability, is easily delivered into cells, and has high-efficiency in vivo protein targeted degradation activity; and effectively solves the problems that the existing proteolysis-targeting chimera molecules are unlikely to achieve transmembrane delivery and degradation of a protein of interest in vivo.

### Solution for solving the problems

In a first aspect, the present disclosure provides a circular RNA molecule, wherein the circular RNA molecule encodes a recombinant polypeptide having a targeting effect activity which is an activity of targeted degradation of a protein of interest.

In some embodiments, according to the circular RNA molecule provided by the present disclosure, the recombinant polypeptide includes a first polypeptide that binds to the protein of interest in a targeted manner and a second polypeptide fused with the first polypeptide, and the second polypeptide has an enzyme activity of a E3 ubiquitin ligase or E3 ligase-binding properties acting as a E3 ligase adaptor or assembly with other proteins to form a E3 ligase complex; optionally, the recombinant polypeptide further includes a linker peptide which links the first polypeptide with the second polypeptide.

In some embodiments, according to the circular RNA molecule provided by the present disclosure, the protein of interest is a transmembrane protein or an intracellular protein; preferably, the protein of interest is a disease-associated protein; and optionally, the disease-associated protein is a tumor-associated protein.

In some alternative embodiments, the tumor-associated protein includes, but is not limited to, K-ras, c-myc, EGFR, PD-L1, PDGF-β, bcl-2, ErbB2, P21, an EGF receptor, RAS, neu, raf, PCNA.

In some embodiments, according to the circular RNA molecule provided by the present disclosure, the first polypeptide is an antibody, antibody fragment or antigen-binding polypeptide that binds to the protein of interest in a targeted manner.

In some embodiments, according to the circular RNA molecule provided by the present disclosure, the second polypeptide is selected from a E3 ubiquitin ligase or a functional fragment thereof; and optionally, the functional fragment of the E3 ubiquitin ligase includes any one of the following: a HECT domain, a RING domain or a U-box domain.

In some embodiments, according to the circular RNA molecule provided by the present disclosure, the second polypeptide is selected from CRBN, VHL, MDM2, cIAP, βTrCP, FBW7, SPOP, SKP2, DDB2, SOCS2, ASB1, CHIP, or a functional fragment of any one of the above.

In some embodiments, according to the circular RNA molecule provided by the present disclosure, the recombinant polypeptide has an amino acid sequence as shown in any one of SEQ ID NOs: 34-45, or a mutant that is obtained by substitution, repeation, deletion or addition of one or more amino acids in the amino acid sequence as shown in any one of SEQ ID NOs: 34-45 and has the polypeptide activity of the recombinant polypeptide.

In some embodiments, according to the circular RNA molecule provided by the present disclosure, the circular RNA molecule includes a coding region sequence encoding the recombinant polypeptide and an IRES sequence operably linked to the coding region sequence.

In some embodiments, according to the circular RNA molecule provided by the present disclosure, the circular RNA molecule further includes a second exon sequence located upstream of the IRES sequence.

In some embodiments, according to the circular RNA molecule provided by the present disclosure, the circular RNA molecule further includes a first exon sequence located downstream of the coding region sequence.

In some embodiments, according to the circular RNA molecule provided by the present disclosure, the circular RNA molecule includes a 5' spacer and a 3' spacer, wherein the 5' spacer sequence is located between the second exon E2 sequence and the IRES sequence, and the 3' spacer sequence is located between the first exon E1 sequence and the coding region sequence.

In some embodiments, according to the circular RNA molecule provided by the present disclosure, the circular RNA molecule includes the following sequences connected sequentially: the second exon sequence, the 5' spacer sequence, the IRES sequence, the coding region sequence, the 3' spacer sequence and the first exon sequence.

In some embodiments, according to the circular RNA molecule provided by the present disclosure, the circular RNA molecule is selected from any one of the group consisting of (i)-(ii):
(i) a nucleotide sequence shown in any one of SEQ ID NO: 2, SEQ ID NO: 4 and SEQ ID NOs: 46-55; and
(ii) a sequence having at least 90%, optionally at least 95%, preferably at least 97%, more preferably at least 98%, and most preferably at least 99% sequence identity with the nucleotide sequence shown in (i).

In some embodiments, according to the circular RNA molecule provided by the present disclosure, the targeting effect activity is an activity of targeted degradation of a protein of interest in a subject; preferably, the circular RNA molecule is administered into the subject; and
optionally, the circular RNA molecule is administered orally, intraperitoneally, intravenously, intra-arterially, intramuscularly, intradermally, subcutaneously, transdermally, nasally, rectally, by intratumoral injection, by tumor intraluminal indwelling, by intrathecal injection, by subarachnoid cavity injection or systemically, and preferably by intratumoral injection.

In a second aspect, the present disclosure provides a cyclization precursor RNA molecule, which is cyclized to form the circular RNA molecule according to the first aspect of the present disclosure; and
optionally, the cyclization precursor RNA molecule includes the following sequences connected sequentially:
a 5' homologous arm sequence, a 3' intron sequence, a second exon sequence, a 5' spacer sequence, an IRES sequence, a coding region sequence, a 3' spacer sequence, a first exon sequence, a 5' intron sequence and a 3' homologous arm sequence.

In a third aspect, the present disclosure provides a recombinant nucleic acid molecule, which is transcribed to form the cyclization precursor RNA molecule according to the present disclosure.

In a fourth aspect, the present disclosure provides a recombinant expression vector, including the recombinant nucleic acid molecule according to the present disclosure.

In a fifth aspect, the present disclosure provides a recombinant host cell, including the circular RNA molecule according to the first aspect of the present disclosure, the cyclization precursor RNA molecule according to the second aspect of the present disclosure, the recombinant nucleic acid molecule according to the third aspect of the present disclosure, or the recombinant expression vector according to the fourth aspect of the present disclosure.

In a sixth aspect, the present disclosure provides a composition, including the circular RNA molecule according to the first aspect of the present disclosure; and optionally further including one or more pharmaceutically acceptable carriers.

In a seventh aspect, the present disclosure provides use of the circular RNA molecule according to the first aspect of the present disclosure, the cyclization precursor RNA molecule according to the second aspect of the present disclosure, the recombinant nucleic acid molecule according to the third aspect of the present disclosure, the recombinant expression vector according to the fourth aspect of the present disclosure, the recombinant host cell according to the fifth aspect of the present disclosure or the composition according to the sixth aspect of the present disclosure in at least one of (a) to (d):
(a) targeted degradation of a protein of interest, preferably targeted degradation of a protein of interest in a subject;
(b) reducing or eliminating the expression of a protein of interest, preferably reducing or eliminating the expression of a protein of interest in a subject;
(c) acting as or preparing an agent for targeted degradation of a protein of interest, preferably acting as or preparing an agent for targeted degradation of a protein of interest in a subject; and
(d) acting as or preparing a drug for treating a disease, preferably acting as or preparing a drug for treating tumor.

In an eighth aspect, the present disclosure provides a method for preventing or treating a disease, including administering the circular RNA molecule according to the first aspect of the present disclosure, the cyclization precursor RNA molecule according to the second aspect of the present disclosure, the recombinant nucleic acid molecule according to the third aspect of the present disclosure, the recombinant expression vector according to the fourth aspect of the present disclosure, the recombinant host cell according to the fifth aspect of the present disclosure or the composition according to the sixth aspect of the present disclosure to a subject;
wherein optionally, the administration is selected from oral administration, intraperitoneal administration, intravenous administration, intra-arterial administration, intramuscular administration, intradermal administration, subcutaneous administration, transdermal administration, nasal administration, rectal administration, intratumoral injection, tumor intraluminal indwelling, intrathecal injection, subarachnoid cavity injection or systemic administration, preferably intratumoral injection.

### Effects of the present invention

In some embodiments, the circular RNA molecule provided by the present disclosure realizes the targeted degradation of the protein of interest for the first time, and provides a novel functional molecule for the technology of targeted degradation of proteins. The circular RNA molecule has good permeability of membranes of tumor cells, is easily delivered into cells, and will not cause metabolic burden of the cells, which effectively solves the problems of difficult transmembrane delivery and low protein degradation activity of the existing proteolysis-targeting chimera molecules.

In some embodiments, the circular RNA molecule provided by the present disclosure, which encodes the second polypeptide having the enzyme activity of the E3 ubiquitin ligase or ligase-binding properties as the E3 ligase adaptor or assembly with other proteins to form the E3 ligase complex, relieves the dependence on the intrinsic ubiquitin ligase in the cells and effectively improves the degradation efficiency of the protein of interest.

In some embodiments, compared with a linear mRNA molecule, the circular RNA molecule provided by the present disclosure has a better protein translation level, protein expression persistence, and more excellent effects of degradation of the protein of interest; and the circular RNA molecule does not need to be capped, subjected to addition of a poly(A) tail, subjected to nucleotide modification, etc., so the production process is simple and the production cost is reduced.

In some preferred embodiments, the circular RNA molecule is suitable for delivery into the body of the subject, which can achieve effective degradation of the protein of interest in the body of the subject, and solve the problems of difficult *in vivo* delivery and low protein degradation activity of the proteolysis-targeting chimera.

In some embodiments, the circular RNA molecule provided by the present disclosure successfully inhibits tumor growth by intratumor injection into tumor model mice, which proves the *in vivo* protein degradation activity of the bio-PROTACs for the first time, and provides a positive and effective treatment solution for the treatment of diseases such as tumor.

### BRIEF DESCRIPTION OF THE DRAWINGS

FIG. 1 shows fluorescence microscopic images of GFP expression 48 hours after cell transfection. A-D in FIG. 1 sequentially represent the fluorescence detection results of co-transfection with EV29-eGFP-DASP mRNA and EV29-H2B-eGFP mRNA of which the amount is 50 ng, 100 ng, 200 ng and 400 ng, respectively.
FIG. 2 shows flow cytometry results 24 hours and 48 hours after cell transfection. In the figure, A represents a flow detection diagram after 24 h, and B represents a flow detection diagram after 48 h.
FIG. 3 shows a comparison of the protein degradation effects of EH2B-eGFP mediated by circular mRNA and linear mRNA.
FIG. 4 shows the expression level of PCNA protein detected by Western Blot.
FIG. 5 shows a flow cytometry result of eGFP expression in tumor cells. In the figure, A represents a proportion of GFP-positive cells in tumor, and B represents the median fluorescence intensity of GFP in the tumor.
FIG. 6 shows a growth curve of the tumor.
FIG. 7 is a diagram showing the detection results of targeted degradation of a KRAS protein by circular RNA molecules.

### DETAILED DESCRIPTION

When used in conjunction with the term "including/comprising" in the claims and/or the specification, the word "a" or "an" can refer to "one", but can also refer to "one or more", "at least one" and "one or more".

As used in the claims and the specification, the words "including", "having", "comprising" or "containing" are meant to be inclusive or open-ended, without excluding additional, unquoted elements or method steps.

Throughout the application document, the term "about" represents that one value includes the standard deviation of an error of a device or method used for determining the value.

Although the disclosure supports the definition of the term "or" as only a substitute and "and/or", the term "or" in the claims refers to "and/or" unless it is explicitly stated that there is only the substitute or substitutes are mutually exclusive.

As used in the present disclosure, terms "polypeptide", "peptide" and "protein" are used interchangeably herein and are amino acid polymers of any length. The polymer may be linear or branched, it may contain modified amino acids, and it may be interrupted by non-amino acids. The term also includes amino acid polymers that have been modified (for example, disulfide bond formation, glycosylation, lipidation, acetylation, phosphorylation, or any other operation, such as conjugation with a labeling component). A polypeptide can be isolated from a natural source, can be produced from a eukaryotic or prokaryotic host by recombinant techniques, and can be a product of a synthetic method.

As used in the present disclosure, a "recombinant polypeptide", also known as "engineered polypeptide", refers to a polypeptide or protein with peptide sequences that are not connected in nature. The recombinant polypeptide can be a polypeptide of interest obtained in prokaryotic or eukaryotic cells by a genetic engineering technology; and can also be a polypeptide of interest obtained by a synthetic technology.

As used in the present disclosure, the term "linker peptide" refers to a linker peptide composed of amino acids, which is used for linking polypeptides, protein or fragments thereof together. In the present disclosure, terms "linker" and "linker peptide" can be used interchangeably.

As used in the present disclosure, the term "E3 ubiquitin ligase", also known as ubiquitin-protein ligase, ubiquitin ligase E3, and E3, has the ability to specifically recognize a protein of interest. The E3 ubiquitin ligase mediates the transfer of ubiquitin from E2 to the protein of interest and covalently binds to the protein of interest by recruiting a substrate protein and a E2 ubiquitin-binding enzyme, and a proteasome catalyzes degradation of a conjugate of ubiquitin and the protein of interest. Currently, a large number of E3 ubiquitin ligases have been discovered, mainly including E3 ubiquitin ligases with a HECT domain, a RING domain or a U-box domain.

As used in the present disclosure, the terms "protein of interest" and "target protein" can be used interchangeably.

As used in the present disclosure, the term "disease-associated protein" refers to a protein abnormally expressed in a diseased individual. Further, the "disease-associated protein" in the present disclosure refers to a protein abnormally highly expressed in a diseased individual.

As used in the present disclosure, the term "antibody" refers to an immunoglobulin or a fragment thereof or their derivatives, and includes any polypeptide with the antigen-binding site it contains, regardless of whether it is produced *in vitro* or *in vivo.* The term includes, but is not limited to, polyclonal, monoclonal, monospecific, multispecific, nonspecific, humanized, single-stranded, chimeric, synthetic, recombinant, hybrid, mutated, grafted antibodies. The term "antibody" also includes antibody fragments such as Fab, F(ab')₂, FV, scFv, Fd, dAb, and other antibody fragments that retain the antigen-binding function. Generally, such fragments will include antigen-binding fragments.

As used in the present disclosure, the term "circular RNA" is a circular RNA molecule in a closed form, which is mainly composed of an exon, an IRES element, a protein coding region and a spacer. In some preferred embodiments, the circular RNA has the following structure: " second exon E2-spacer-IRES element-coding region-spacer-first exon E1". The circular RNA used in the present disclosure has protein translation activity, and can also be called "circular mRNA".

As used in the present disclosure, the term "cyclization precursor RNA" refers to an RNA precursor that is capable of being cyclized to form circular RNA, and is generally formed by transcription of a DNA molecule of a cyclization precursor. In some embodiments, the following elements are included in the cyclization precursor RNA: a 5' homologous arm, a 3' intron, a second exon E2, a spacer, an IRES element, a coding region, a spacer, a first exon E1, a 5' intron and a 3' homologous arm. In some embodiments, the 5' homologous arm, the 3' intron, the 5' intron and the 3' homologous arm play roles in the process of cleavage of the cyclization precursor RNA to form the circular RNA. Wherein, the intron has ribozyme characteristics. For example, by using the ribozyme characteristics of the 5' intron, the connection between the 5' intron and the first exon E1 is broken under the trigger of GTP, and the broken site can further cause the connection between the 3' intron and the second exon E2 to be broken, and then the second exon E2 is connected with the first exon E1 to form the circular RNA.

As used in the present disclosure, the term "linear RNA" refers to RNA having a translation function, including a 5' cap, a 3' PolyA tail, a 5' untranslated region (5'UTR), a 3' untranslated region (3'UTR), an open reading frame (ORF) and the like structures.

As used in the present disclosure, the term "IRES" is also called an internal ribosome entry site. The "internal ribosome entry site" (IRES) belongs to a translation control sequence, generally located at the 5' terminal of the gene of interest, and enables RNA to be translated in a cap-independent manner. The transcribed IRES can directly bind to a ribosome subunit, so that a start codon of mRNA is properly oriented in the ribosome for translation. The IRES sequence is usually located in the 5'UTR of the mRNA (just upstream of the start codon). IRES functionally replaces the demands for various protein factors that interact with the translation mechanism of eukaryotes.

As used in the present disclosure, the term "coding region" refers to a gene sequence that can transcribe a messenger RNA and finally translate it into a polypeptide or protein of interest.

As used in the present disclosure, the term "expression" includes any step involving the production of a polypeptide, including but not limited to: transcription, post-transcriptional modification, translation, post-translational modification, and secretion.

As used in the present disclosure, the term "upstream" or "downstream" refers to the upstream and downstream along a protein translation direction of a coding region.

As used in the present disclosure, the terms "polynucleotide" and "nucleic acid molecule" refer to polymers composed of nucleotides. Polynucleotide can be in the form of an individual fragment or an integral part of a larger nucleotide sequence structure. It is derived from a nucleotide sequence that has been separated at least once in number or concentration, and can recognize, manipulate and restore a sequence and a component nucleotide sequence thereof by a standard molecular biology method (e.g., using cloning vectors). When a nucleotide sequence is represented by a DNA sequence (i.e. A, T, G, C), this also includes an RNA sequence (i.e. A, U, G, C), where "U" replaces "T". In other words, the "polynucleotide" refers to a nucleotide polymer that has been removed from other nucleotides (individual fragments or an entire fragment), or may be an integral part or component of a larger nucleotide structure, such as an expression vector or a polycistronic sequence. The polynucleotide includes DNA, RNA and cDNA sequences. The "recombinant polynucleotide" and the "recombinant nucleic acid molecule" belong to one of the "polynucleotides".

As used in the present disclosure, the term "recombinant nucleic acid molecule" refers to a polynucleotide with sequences that are not linked together in nature. The recombinant polynucleotide can be included in a suitable vector, and the vector can be used for transformation into suitable host cells. Then the polynucleotide is expressed in recombinant host cells to produce, for example, the "recombinant polypeptide", "recombinant protein", "fusion protein", and the like.

As used in the present disclosure, the term "vector" refers to a DNA construct, which contains a DNA sequence operably linked to a suitable control sequence, so as to express the gene of interest in a suitable host.

As used in the present disclosure, the term "recombinant expression vector" refers to a DNA structure used for expressing, for example, a polynucleotide encoding a desired polypeptide. The recombinant expression vector may include, for example, i) a set of genetic elements that have regulatory effects on gene expression, such as promoters and enhancers; ii) a structure or coding sequence that is transcribed into mRNA and translated into a protein; and iii) a transcription subunit that appropriately transcripts and translates initiation and termination sequences. The recombinant expression vector is constructed in any suitable way. The nature of the vector is not critical, and any vector can be used, including a plasmid, a virus, a phage and a transposon. Possible vectors for the present disclosure include, but are not limited to, chromosomal, non-chromosomal and synthetic DNA sequences, such as viral plasmids, bacterial plasmids, phage DNAs, yeast plasmids and vectors derived from a combination of plasmids and phage DNAs, and DNAs from viruses such as lentiviruses, retroviruses, vaccinia viruses, adenoviruses, fowlpox viruses, baculoviruses, SV40 and Pseudorabies viruses.

As used in the present disclosure, the term "host cell" refers to a cell into which an exogenous polynucleotide has been introduced, including progenies of such cells. The host cell includes a "transformant" and a "transformed cell", which include a primary transformed cell and a progeny derived therefrom. The host cell is any type of cell system that can be used for producing the antibody molecule of the present invention, including eukaryotic cells, for example, mammalian cells, insect cells, yeast cells; and prokaryotic cells, for example, *Escherichia coli* cells. The host cell includes a cultured cell, as well as a cell in a transgenic animal or plant, or in a cultured plant or animal tissue. The term "recombinant host cell" covers a host cell which is different from a parent cell after the introduction of the circular RNA, the cyclization precursor RNA, the recombinant nucleic acid molecule or the recombinant expression vector, and the recombinant host cell is specifically realized by transformation. The host cell of the present disclosure can be a prokaryotic or eukaryotic cell, as long as the circular RNA, the cyclization precursor RNA, the recombinant nucleic acid molecule or the recombinant expression vector of the present disclosure can be introduced into it.

As used in the present disclosure, the terms "transformation, transfection, and transduction" have the meaning commonly understood by those skilled in the art, that is, a process of introducing exogenous DNA into a host. The methods of transformation, transfection and transduction include any method of introducing nucleic acid into cells, including but not limited to an electroporation method, a calcium phosphate (CaPO₄) precipitation method, a calcium chloride (CaCl₂) precipitation method, a microinjection method, a polyethylene glycol (PEG) method, a DEAE-dextran method, a cationic liposome method and a lithium acetate-DMSO method.

As used in the present disclosure, "treatment" refers to contacting (e.g., administering) the subject with the circular RNA, the cyclization precursor RNA, the recombinant nucleic acid molecule, the recombinant expression vector, and the composition of the present invention after a subject suffers from a disease, so as to reduce the symptoms of the disease compared with those without contact, which does not mean that it is necessary to completely suppress the symptoms of the disease. Suffering from a disease means that: symptoms of the disease have been occurred in the body.

As used in the present disclosure, "prevention" refers to contacting (e.g., administering) the subject with the circular RNA, the cyclization precursor RNA, the recombinant nucleic acid molecule, the recombinant expression vector, the composition and the like of the present invention before a subject suffers from a disease, so as to reduce the symptoms after the subject suffers from the disease compared with those without contact, which does not mean that it is necessary to completely suppress suffering from the disease.

As used in the present disclosure, the term "effective amount" refers to such amount or dose of the recombinant nucleic acid molecule, the recombinant expression vector, the cyclization precursor RNA, the circular RNA, a vaccine or the composition of the present invention, which produces an expected effect in the patient in need of treatment or prevention after being administered to a patient in single or multiple doses. The effective amount can be easily determined by an attending physician as a person skilled in the art by considering the following various factors: species such as mammals; a size, age and general health thereof; a specific disease involved; the degree or severity of the disease; the response of an individual patient; a specific antibody administered; an administration mode; the bioavailability characteristics of the administrated formulation; a selected administration regimen; and the use of any concomitant therapy.

As used in the present disclosure, the terms "individual", "patient" or "subject" include mammals. The mammals include, but are not limited to, domestic animals (e.g., cattle, sheep, cats, dogs and horses), primates (e.g., human and non-human primates such as monkeys), rabbits, and rodents (e.g., mice and rats).

The terms "cancer" and "cancerous" refer to or describe physiological disorders in mammals which are usually characterized by unregulated cell growth. Examples of cancer include, but are not limited to, cancer, lymphoma, blastoma, sarcoma and leukemia or lymphoid malignant tumor. More specific examples of such cancers include, but are not limited to, squamous cell carcinoma (e.g., epithelial squamous cell carcinoma), lung cancer (including small cell lung cancer, non-small cell lung cancer, adenocarcinoma of lung, and squamous carcinoma of lung), peritoneal cancer, hepatocellular carcinoma, gastric cancer (including gastrointestinal cancer and gastrointestinal stromal cancer), pancreatic cancer, glioblastoma, cervical cancer, ovarian cancer, liver cancer, bladder cancer, urethral carcinoma, hepatoma, breast cancer, colon cancer, rectal cancer, colorectal cancer, endometrial cancer or uterine cancer, salivary gland carcinoma, renal cancer, prostate cancer, vulvar cancer, thyroid cancer, liver cancer, anal cancer, penile cancer, melanoma, superficial diffuse melanoma, lentigo maligna melanoma, acral melanoma, nodular melanoma, multiple myeloma and B-cell lymphoma, chronic lymphocytic leukemia (CLL), acute lymphoblastic leukemia (ALL), hairy cell leukemia, chronic myeloid leukemia, and post-transplant lymphoproliferative disorders (PTLD), as well as abnormal vascular proliferation related to phakomatoses, edema (such as those related to brain tumor) and Meigs's syndrome, brain tumor and brain cancer, head and neck cancer, and related metastasis. In certain embodiments, cancers suitable for treatment by the antibodies of the present invention include lung cancer (e.g., non-small cell lung cancer), liver cancer, stomach cancer or colon cancer, including metastatic forms of those cancers.

The term "tumor" refers to all growth and proliferation of neoplastic cells, whether malignant or benign, and all pre-cancerous and cancerous cells and tissues. The terms "cancer", "cancerous" and "tumor" are not exclusive with each other when mentioned herein.

Unless otherwise defined or clearly indicated by the background, all technical and scientific terms in the present disclosure have the same meanings as commonly understood by those of ordinary skill in the art to which the present disclosure belongs.

### Technical solutions

In the technical solution of the present disclosure, the meanings represented by the numbers of nucleotide and amino acid sequence lists in the specification are as follows:
a sequence shown in SEQ ID NO: 1 is a nucleotide sequence of a circular RNA molecule encoding a H2B-eGFP protein;
a sequence shown in SEQ ID NO: 2 is a nucleotide sequence of a circular RNA molecule encoding a recombinant polypeptide eGFP-DASP;
a sequence shown in SEQ ID NO: 3 is a nucleotide sequence of a linear RNA molecule encoding the recombinant polypeptide eGFP-DASP;
a sequence shown in SEQ ID NO: 4 is a nucleotide sequence of a circular RNA molecule encoding a recombinant polypeptide CON1-SP-WT;
a sequence shown in SEQ ID NO: 5 is a nucleotide sequence of a circular RNA molecule encoding a recombinant polypeptide CON1-SP-mu;
a sequence shown in SEQ ID NO: 6 is a nucleotide sequence of a circular RNA molecule encoding a recombinant polypeptide CON1-mu-SP;
a sequence shown in SEQ ID NO: 7 is an amino acid sequence of the PCNA protein;
a sequence shown in SEQ ID NO: 8 is an amino acid sequence of the first polypeptide that binds to the PCNA protein in a targeted manner;
a sequence shown in SEQ ID NO: 9 is a nucleotide sequence encoding the first polypeptide that binds to the PCNA protein in a targeted manner;
a sequence shown in SEQ ID NO: 10 is an amino acid sequence of the linker peptide connecting the first polypeptide and the second polypeptide;
a sequence shown in SEQ ID NO: 11 is a nucleotide sequence encoding the linker peptide;
a sequence shown in SEQ ID NO: 12 is an amino acid sequence of the second polypeptide with ubiquitin ligase activity of SPOP;
a sequence shown in SEQ ID NO: 13 is a nucleotide sequence encoding the second polypeptide with ubiquitin ligase activity of SPOP;
a sequence shown in SEQ ID NO: 14 is a nucleotide sequence of CVB3 IRES;
a sequence shown in SEQ ID NO: 15 is a nucleotide sequence of EV24 IRES;
a sequence shown in SEQ ID NO: 16 is a nucleotide sequence of EV29 IRES;
a sequence shown in SEQ ID NO: 17 is a nucleotide sequence of EV33 IRES;
a sequence shown in SEQ ID NO: 18 is a nucleotide sequence of IRES chimeric with EV24 and CVB3v;
a sequence shown in SEQ ID NO: 19 is a nucleotide sequence of IRES chimeric with EV29 and CVB3v;
a sequence shown in SEQ ID NO: 20 is a nucleotide sequence of IRES chimeric with EV33 and CVB3v;
a sequence shown in SEQ ID NO: 21 is a nucleotide sequence of the first exon (E1);
a sequence shown in SEQ ID NO: 22 is a nucleotide sequence of the second exon (E2);
a sequence shown in SEQ ID NO: 23 is a nucleotide sequence of the 5' spacer sequence 1;
a sequence shown in SEQ ID NO: 24 is a nucleotide sequence of the 5' spacer sequence 2;
a sequence shown in SEQ ID NO: 25 is a nucleotide sequence of the 3' spacer sequence 1;
a sequence shown in SEQ ID NO: 26 is a nucleotide sequence of the 3' spacer sequence 2;
a sequence shown in SEQ ID NO: 27 is a nucleotide sequence of the 5' intron;
a sequence shown in SEQ ID NO: 28 is a nucleotide sequence of the 3' intron;
a sequence shown in SEQ ID NO: 29 is a nucleotide sequence of the 5' homologous arm sequence 1 (H1);
a sequence shown in SEQ ID NO: 30 is a nucleotide sequence of the 5' homologous arm sequence 2 (H2);
a sequence shown in SEQ ID NO: 31 is a nucleotide sequence of the 3' homologous arm sequence 1;
a sequence shown in SEQ ID NO: 32 is a nucleotide sequence of the 3' homologous arm sequence 2;
a sequence shown in SEQ ID NO: 33 is an amino acid sequence of K19/K19-VHKC targeting the KRAS protein;
a sequence shown in SEQ ID NO: 34 is an amino acid sequence of a recombinant polypeptide N-eGFP (first polypeptide)-linker peptide-SPOP (second polypeptide)-C;
a sequence shown in SEQ ID NO: 35 is an amino acid sequence of a recombinant polypeptide N-CON1 (first polypeptide)-linker peptide-SPOP (second polypeptide)-C;
a sequence shown in SEQ ID NO: 36 is an amino acid sequence of a recombinant polypeptide N-βTrCP (second polypeptide)-linker peptide-K19 (first polypeptide)-C;
a sequence shown in SEQ ID NO: 37 is an amino acid sequence of a recombinant polypeptide N-FBW7 (second polypeptide)-linker peptide-K19 (first polypeptide)-C;
a sequence shown in SEQ ID NO: 38 is an amino acid sequence of a recombinant polypeptide N-SKP2 (second polypeptide)-linker peptide-K19 (first polypeptide)-C;
a sequence shown in SEQ ID NO: 39 is an amino acid sequence of a recombinant polypeptide N-CRBN (second polypeptide)-linker peptide-K19 (first polypeptide)-C;
a sequence shown in SEQ ID NO: 40 is an amino acid sequence of a recombinant polypeptide N-DDB2 (second polypeptide)-linker peptide-K19 (first polypeptide)-C;
a sequence shown in SEQ ID NO: 41 is an amino acid sequence of a recombinant polypeptide N-K19 (first polypeptide)-linker peptide-SOCS2 (second polypeptide)-C;
a sequence shown in SEQ ID NO: 42 is an amino acid sequence of a recombinant polypeptide N-K19 (first polypeptide)-linker peptide-ASB1 (second polypeptide)-C;
a sequence shown in SEQ ID NO: 43 is an amino acid sequence of a recombinant polypeptide N-K19 (first polypeptide)-linker peptide-CHIP (second polypeptide)-C;
a sequence shown in SEQ ID NO: 44 is an amino acid sequence of a recombinant polypeptide N-K19 (first polypeptide)-linker peptide-SPOP (second polypeptide)-C;
a sequence shown in SEQ ID NO: 45 is an amino acid sequence of a recombinant polypeptide N-K19 (first polypeptide)-linker peptide-VHL (second polypeptide)-C;
a sequence shown in SEQ ID NO: 46 is a nucleotide sequence of a circular RNA molecule encoding a recombinant polypeptide N-βTrCP (second polypeptide)-linker peptide-K19 (first polypeptide)-C;
a sequence shown in SEQ ID NO: 47 is a nucleotide sequence of a circular RNA molecule encoding a recombinant polypeptide N-FBW7 (second polypeptide)-linker peptide-K19 (first polypeptide)-C;
a sequence shown in SEQ ID NO: 48 is a nucleotide sequence of a circular RNA molecule encoding a recombinant polypeptide N-SKP2 (second polypeptide)-linker peptide-K19 (first polypeptide)-C;
a sequence shown in SEQ ID NO: 49 is a nucleotide sequence of a circular RNA molecule encoding a recombinant polypeptide N-CRBN (second polypeptide)-linker peptide-K19 (first polypeptide)-C;
a sequence shown in SEQ ID NO: 50 is a nucleotide sequence of a circular RNA molecule encoding a recombinant polypeptide N-DDB2 (second polypeptide)-linker peptide-K19 (first polypeptide)-C;
a sequence shown in SEQ ID NO: 51 is a nucleotide sequence of a circular RNA molecule encoding a recombinant polypeptide N-K19 (first polypeptide)-linker peptide-SOCS2 (second polypeptide)-C;
a sequence shown in SEQ ID NO: 52 is a nucleotide sequence of a circular RNA molecule encoding a recombinant polypeptide N-K19 (first polypeptide)-linker peptide-ASB1 (second polypeptide)-C;
a sequence shown in SEQ ID NO: 53 is a nucleotide sequence of a circular RNA molecule encoding a recombinant polypeptide N-K19 (first polypeptide)-linker peptide-CHIP (second polypeptide)-C;
a sequence shown in SEQ ID NO: 54 is a nucleotide sequence of a circular RNA molecule encoding a recombinant polypeptide N-K19 (first polypeptide)-linker peptide-SPOP (second polypeptide)-C;
a sequence shown in SEQ ID NO: 55 is a nucleotide sequence of a circular RNA molecule encoding a recombinant polypeptide N-K19 (first polypeptide)-linker peptide-VHL (second polypeptide)-C;
a sequence shown in SEQ ID NO: 56 is a nucleotide sequence of a circular RNA molecule EV29-K19-VHKC;
a sequence shown in SEQ ID NO: 57 is an amino acid sequence of the linker peptide.

### Circular RNA molecule

The circular RNA molecule of the present disclosure encodes a recombinant polypeptide having a targeting effect activity which is an activity of targeted degradation of a protein of interest. The circular RNA molecule can realize the specific degradation of the protein of interest by expressing a recombinant polypeptide capable of degrading the protein of interest in a targeted manner, and provides a novel functional molecule for a technology of targeted degradation of proteins.

Those skilled in the art know that the proteolysis-targeting chimera in a small molecular form has poor membrane permeability and is difficult to deliver into cells to play a role in protein degradation; while the Bio-PROTAC molecule in the form of chimeric protein has a large molecular weight, and thus there are larger technical difficulties in *in vivo* transmembrane delivery. Therefore, currently, the proteolysis-targeting chimera generally has the problems of difficult *in vivo* delivery and difficulty of effectively degrading the protein of interest. Compared with this, the circular RNA molecule in the present disclosure has good membrane permeability, and is easily delivered into cells, especially into the body, so as to achieve efficient and specific degradation of the protein of interest.

In some embodiments, the recombinant polypeptide includes a first polypeptide that binds to the protein of interest in a targeted manner and a second polypeptide fused with the first polypeptide, and the second polypeptide has an enzyme activity of a E3 ubiquitin ligase. By fusing the first polypeptide and the second polypeptide to form the recombinant polypeptide, after targeted binding to the protein of interest, the protein of interest can be ubiquitinated by the enzyme activity of the E3 ubiquitin ligase, and the ubiquitinated protein of interest is recognized and degraded by a proteasome.

In the present disclosure, the protein of interest can be of any protein type involved in physiological and pathological processes of the body, including a transmembrane protein and an intracellular protein.

In some embodiments, the protein of interest is a disease-associated protein. Disease types include, but are not limited to, tumors, nervous system diseases, immune system diseases, cardiovascular and cerebrovascular diseases, etc. In some embodiments, the disease-associated protein is a protein that is abnormally expressed in any type of disease. Further, the disease-associated protein is a protein abnormally highly expressed in the process of occurrence, development and prognosis of the disease. In the present disclosure, "abnormally highly expressed" means that the protein expression level is over-expression compared with that of a healthy individual who is not diseased. The circular RNA molecule can achieve the effect of disease treatment by specifically and efficiently degrading the disease-associated protein.

In some embodiments, the protein of interest is a protein involved in physiological activities of the body. Exemplarily, the protein of interest is a protein involved in the regulation of life activities such as cell cycle, proliferation, apoptosis, differentiation and metastasis. By utilizing the circular RNA molecule of the present disclosure, targeted degradation of the protein of interest can be realized, which provides a foundation for the research of the protein function. In the present disclosure, the protein involved in physiological activities of the body and the disease-associated protein are not exclusive with each other.

In some more specific embodiments, the protein of interest is a tumor-associated protein. Exemplarily, the protein of interest includes, but is not limited to, K-ras, c-myc, EGFR, PD-L1, PDGF-β, bcl-2, ErbB2, P21, an EGF receptor, RAS, neu, raf, PCNA, etc.

In some alternative embodiments, the protein of interest is PCNA. The amino acid sequence of PCNA for example includes the amino acid sequence shown in SEQ ID NO: 7, or a protein mutant that is obtained by substitution, repeation, deletion or addition of one or more amino acids in the amino acid sequence shown in SEQ ID NO: 7 and has the PCNA protein activity.

PCNA plays a role in cellular activities such as DNA replication, DNA excision and repair, cell cycle regulation, chromatin assembly, RNA transcription and the like, can be used as a standard marker of tumor proliferation activity to evaluate the division and proliferation of tumor cells, is highly expressed in many malignant tumors, and is a potential drug target. In the present disclosure, the tumor growth is effectively inhibited by intratumor injection of the circular RNA molecule that degrades PCNA in a targeted manner into the tumor model mice, showing that the circular RNA molecule of the present disclosure is suitable for delivery in the body of a subject with high delivery efficiency, has obvious protein targeted degradation effects, and has a wide application prospect in disease treatment.

In some alternative embodiments, the protein of interest is KRAS. When KRAS is abnormally highly expressed, the continuous growth of cells is caused and the cells are prevented from self-destruction, thereby making the cells proliferate out of control and become cancerous.

In the present disclosure, the first polypeptide does not have a particularly defined sequence, and its specific sequence is designed according to a sequence of the protein of interest to be bound.

In some embodiments, the first polypeptide is an antibody, antibody fragment or antigen-binding polypeptide that binds to the protein of interest in a targeted manner. Exemplarily, the first polypeptide may be a natural antibody that binds to the protein of interest, or an antibody fragment prepared by a recombinant DNA technology or by enzymatic or chemical cleavage of an intact antibody, or an antigen-binding polypeptide that binds to an antigen.

In the present disclosure, the antibody refers to a protein containing an antigen-binding site, encompasses natural antibodies and artificial antibodies of various structures, including but not limited to monoclonal antibodies, polyclonal antibodies, multispecific antibodies (e.g., bispecific antibodies), single chain antibodies, intact antibodies, etc.

In the present disclosure, the antibody fragment includes a part of an intact antibody and binds to an antigen to which the intact antibody binds. Examples of the antibody fragment include, but are not limited to Fv, Fab, Fab', Fab'-SH, and F(ab')₂; a double antibody; a linear antibody; a single chain antibody (e.g. ScFv); a single domain antibody; a bivalent or bispecific antibody or a fragment thereof; a camelidae antibody; and a bispecific or multispecific antibody formed by antibody fragments.

In the present disclosure, the antigen-binding polypeptide can be a chemically synthesized or recombinantly prepared polypeptide or polypeptide fragment that specifically binds to the protein of interest.

Exemplarily, the first polypeptide is DARPins, αReps, a single domain antibody, a binding peptide, etc. that bind to the protein of interest.

In some alternative embodiments, the first polypeptide binds to PCNA in a targeted manner, and the amino acid sequence of the first polypeptide includes the amino acid sequence as shown in SEQ ID NO: 8, or a mutant that is obtained by substitution, repeation, deletion or addition of one or more amino acids in the amino acid sequence shown in SEQ ID NO: 8 and has the protein activity of binding to PCNA in a targeted manner.

In some alternative embodiments, the coding region sequence that encodes the first polypeptide includes the nucleotide sequence shown in SEQ ID NO: 9, or a sequence having 80%, 81%, 82%, 83%, 84%, 85%, 86%, 87%, 88%, 89%, 90%, 91%, 92%, 93%, 94%, 95%, 96%, 97%, 98% and 99% sequence identity with the nucleotide sequence shown in SEQ ID NO: 9, and encodes a protein mutant with the protein activity of binding to PCNA in a targeted manner.

In some alternative embodiments, the first polypeptide binds to KRAS in a targeted manner, and the amino acid sequence of the first polypeptide includes the amino acid sequence shown in SEQ ID NO: 33, or a mutant that is obtained by substitution, repeation, deletion or addition of one or more amino acids in the amino acid sequence shown in SEQ ID NO: 33 and has the protein activity of binding to KRAS in a targeted manner.

In the present disclosure, the second polypeptide does not have a particularly defined sequence, as long as it can make the second polypeptide have an enzyme activity of a E3 ubiquitin ligase or E3 ligase-binding properties acting as a E3 ligase adaptor or assembly with other proteins to form a E3 ligase complex, so as to realize ubiquitination labeling of the protein of interest.

In some embodiments, the second polypeptide is selected from the E3 ubiquitin ligase or a functional fragment thereof, and specifically, the second polypeptide can be selected from any E3 ubiquitin ligase with the ubiquitin ligase activity or a functional fragment thereof. Further, the functional fragment of the E3 ubiquitin ligase includes any one of the following: a HECT domain, a RING domain or a U-box domain. Wherein, a conserved Cys residue of the HECT domain can form a thioester bond with ubiquitin carried by E2, and the ubiquitin is first transmitted by E2 to E3, and then presented to a substrate by E3. The RING domain includes a similar E2 binding domain, which acts as a bridge to directly transfer the activated ubiquitin from E2 to the protein of interest. The U-box domain is linked to the ubiquitin binding enzyme E2, then forms an intermediate complex with the ubiquitin through the thioester bond, and finally catalyzes the polyubiquitination modification of the protein of interest.

In some alternative embodiments, the second polypeptide is selected from any one of the following E3 ubiquitin ligases or functional fragments thereof: CRBN, VHL, MDM2, cIAP, βTrCP, FBW7, SPOP, SKP2, DDB2, SOCS2, ASB1, and CHIP.

In some alternative embodiments, the second polypeptide is a SPOP protein domain with E3 ubiquitin ligase activity, and its amino acid sequence includes the amino acid sequence shown in SEQ ID NO: 12, or a mutant that is obtained by substitution, repeation, deletion or addition of one or more amino acids in the amino acid sequence shown in SEQ ID NO: 12 and has the E3 ubiquitin ligase activity.

In some alternative embodiments, the coding region sequence that encodes the second polypeptide includes the nucleotide sequence shown in SEQ ID NO: 13, or a sequence having 80%, 81%, 82%, 83%, 84%, 85%, 86%, 87%, 88%, 89%, 90%, 91%, 92%, 93%, 94%, 95%, 96%, 97%, 98% and 99% sequence identity with the nucleotide sequence shown in SEQ ID NO: 13, and encodes a protein mutant with the E3 ubiquitin ligase activity.

In some other embodiments, the second polypeptide may also be a protein domain of βTrCP, FBW7, SKP2, CRBN, DDB2, SOCS2, ASB1, CHIP, SPOP, VHL, etc. with the E3 ubiquitin ligase activity.

In some embodiments, the recombinant polypeptide further includes a linker peptide which links the first polypeptide with the second polypeptide. The first polypeptide is linked with the second polypeptide by the linker peptide, so as to optimize the spatial conformation of the recombinant polypeptide and ensure the protein activity of binding to the protein of interest in a targeted manner and the enzyme activity of the E3 ubiquitin ligase.

In some alternative embodiments, the amino acid sequence of the linker peptide includes the amino acid sequence shown in SEQ ID NO: 10, or a mutant that is obtained by substitution, repeat, deletion or addition of one or more amino acids in the amino acid sequence shown in SEQ ID NO: 10 or 57 and has the linker peptide activity.

In some alternative embodiments, the coding region sequence that encodes the linker peptide shown in SEQ ID NO: 10 includes the nucleotide sequence shown in SEQ ID NO: 11, or a sequence having 80%, 81%, 82%, 83%, 84%, 85%, 86%, 87%, 88%, 89%, 90%, 91%, 92%, 93%, 94%, 95%, 96%, 97%, 98% and 99% sequence identity with the nucleotide sequence shown in SEQ ID NO: 11, and encodes a protein mutant with the linker peptide activity.

In some specific embodiments, the recombinant polypeptide has a structure shown in any one of the following:
(1) N-eGFP (first polypeptide)-linker peptide-SPOP (second polypeptide)-C;
(2) N-CON1 (first polypeptide)-linker peptide-SPOP (second polypeptide)-C;
(3) N-βTrCP (second polypeptide)-linker peptide-K19 (first polypeptide)-C;
(4) N-FBW7 (second polypeptide)-linker peptide-K19 (first polypeptide)-C;
(5) N-SKP2 (second polypeptide)-linker peptide-K19 (first polypeptide)-C;
(6) N-CRBN (second polypeptide)-linker peptide-K19 (first polypeptide)-C;
(7) N-DDB2 (second polypeptide)-linker peptide-K19 (first polypeptide)-C;
(8) N-K19 (first polypeptide)-linker peptide-SOCS2 (second polypeptide)-C;
(9) N-K19 (first polypeptide)-linker peptide-ASB1 (second polypeptide)-C;
(10) N-K19 (first polypeptide)-linker peptide-CHIP (second polypeptide)-C;
(11) N-K19 (first polypeptide)-linker peptide-SPOP (second polypeptide)-C;
(12) N-K19 (first polypeptide)-linker peptide-VHL (second polypeptide)-C,
wherein the recombinant polypeptide with the structure shown in (1) has the amino acid sequence shown in SEQ ID NO: 34, or a mutant that is obtained by substitution, repeation, deletion or addition of one or more amino acids in the amino acid sequence shown in SEQ ID NO: 34 and has the activity of the polypeptide shown in SEQ ID NO: 34. The recombinant polypeptide utilizes the first polypeptide to bind to an eGFP protein in a targeted manner, and then utilizes the E3 ubiquitin ligase activity of the second polypeptide to realize the targeted degradation of the eGFP protein.

The recombinant polypeptide with the structure shown in (2) has the amino acid sequence shown in SEQ ID NO: 35, or a mutant that is obtained by substitution, repeation, deletion or addition of one or more amino acids in the amino acid sequence shown in SEQ ID NO: 35 and has the activity of the polypeptide shown in SEQ ID NO: 35. The recombinant polypeptide utilizes the first polypeptide to bind to the PCNA protein in a targeted manner, and then utilizes the E3 ubiquitin ligase activity of the second polypeptide to realize the targeted degradation of the PCNA protein.

The recombinant polypeptide with the structure shown in (3) has the amino acid sequence shown in SEQ ID NO: 36, or a mutant that is obtained by substitution, repeation, deletion or addition of one or more amino acids in the amino acid sequence shown in SEQ ID NO: 36 and has the activity of the polypeptide shown in SEQ ID NO: 36. The recombinant polypeptide utilizes the first polypeptide to bind to the KRAS protein in a targeted manner, and then utilizes the E3 ubiquitin ligase activity of the second polypeptide to realize the targeted degradation of the KRAS protein.

The recombinant polypeptide with the structure shown in (4) has the amino acid sequence shown in SEQ ID NO: 37, or a mutant that is obtained by substitution, repeation, deletion or addition of one or more amino acids in the amino acid sequence shown in SEQ ID NO: 37 and has the activity of the polypeptide shown in SEQ ID NO: 37. The recombinant polypeptide utilizes the first polypeptide to bind to the KRAS protein in a targeted manner, and then utilizes the E3 ubiquitin ligase activity of the second polypeptide to realize the targeted degradation of the KRAS protein.

The recombinant polypeptide with the structure shown in (5) has the amino acid sequence shown in SEQ ID NO: 38, or a mutant that is obtained by substitution, repeation, deletion or addition of one or more amino acids in the amino acid sequence shown in SEQ ID NO: 38 and has the activity of the polypeptide shown in SEQ ID NO: 38. The recombinant polypeptide utilizes the first polypeptide to bind to the KRAS protein in a targeted manner, and then utilizes the E3 ubiquitin ligase activity of the second polypeptide to realize the targeted degradation of the KRAS protein.

The recombinant polypeptide with the structure shown in (6) has the amino acid sequence shown in SEQ ID NO: 39, or a mutant that is obtained by substitution, repeation, deletion or addition of one or more amino acids in the amino acid sequence shown in SEQ ID NO: 39 and has the activity of the polypeptide shown in SEQ ID NO: 39. The recombinant polypeptide utilizes the first polypeptide to bind to the KRAS protein in a targeted manner, and then utilizes the E3 ubiquitin ligase activity of the second polypeptide to realize the targeted degradation of the KRAS protein.

The recombinant polypeptide with the structure shown in (7) has the amino acid sequence shown in SEQ ID NO: 40, or a mutant that is obtained by substitution, repeation, deletion or addition of one or more amino acids in the amino acid sequence shown in SEQ ID NO: 40 and has the activity of the polypeptide shown in SEQ ID NO: 40. The recombinant polypeptide utilizes the first polypeptide to bind to the KRAS protein in a targeted manner, and then utilizes the E3 ubiquitin ligase activity of the second polypeptide to realize the targeted degradation of the KRAS protein.

The recombinant polypeptide with the structure shown in (8) has the amino acid sequence shown in SEQ ID NO: 41, or a mutant that is obtained by substitution, repeation, deletion or addition of one or more amino acids in the amino acid sequence shown in SEQ ID NO: 41 and has the activity of the polypeptide shown in SEQ ID NO: 41. The recombinant polypeptide utilizes the first polypeptide to bind to the KRAS protein in a targeted manner, and then utilizes the E3 ubiquitin ligase activity of the second polypeptide to realize the targeted degradation of the KRAS protein.

The recombinant polypeptide with the structure shown in (9) has the amino acid sequence shown in SEQ ID NO: 42, or a mutant that is obtained by substitution, repeation, deletion or addition of one or more amino acids in the amino acid sequence shown in SEQ ID NO: 42 and has the activity of the polypeptide shown in SEQ ID NO: 42. The recombinant polypeptide utilizes the first polypeptide to bind to the KRAS protein in a targeted manner, and then utilizes the E3 ubiquitin ligase activity of the second polypeptide to realize the targeted degradation of the KRAS protein.

The recombinant polypeptide with the structure shown in (10) has the amino acid sequence shown in SEQ ID NO: 43, or a mutant that is obtained by substitution, repeation, deletion or addition of one or more amino acids in the amino acid sequence shown in SEQ ID NO: 43 and has the activity of the polypeptide shown in SEQ ID NO: 43. The recombinant polypeptide utilizes the first polypeptide to bind to the KRAS protein in a targeted manner, and then utilizes the E3 ubiquitin ligase activity of the second polypeptide to realize the targeted degradation of the KRAS protein.

The recombinant polypeptide with the structure shown in (11) has the amino acid sequence shown in SEQ ID NO: 44, or a mutant that is obtained by substitution, repeation, deletion or addition of one or more amino acids in the amino acid sequence shown in SEQ ID NO: 44 and has the activity of the polypeptide shown in SEQ ID NO: 44. The recombinant polypeptide utilizes the first polypeptide to bind to the KRAS protein in a targeted manner, and then utilizes the E3 ubiquitin ligase activity of the second polypeptide to realize the targeted degradation of the KRAS protein.

The recombinant polypeptide with the structure shown in (12) has the amino acid sequence shown in SEQ ID NO: 45, or a mutant that is obtained by substitution, repeation, deletion or addition of one or more amino acids in the amino acid sequence shown in SEQ ID NO: 45 and has the activity of the polypeptide shown in SEQ ID NO: 45. The recombinant polypeptide utilizes the first polypeptide to bind to the KRAS protein in a targeted manner, and then utilizes the E3 ubiquitin ligase activity of the second polypeptide to realize the targeted degradation of the KRAS protein.

The circular RNA molecule of the present disclosure effectively solves the problems that it is difficult to realize intracellular delivery of the proteolysis-targeting chimera and the proteolysis-targeting chimera cannot effectively play a role. Encoding a chimera of the first polypeptide and the second polypeptide, in which the second polypeptide has the enzyme activity of the E3 ubiquitin ligase and is not limited by the intracellular E3 ubiquitin ligase, has the advantages of high degradation efficiency and good stability.

In some embodiments, the circular RNA molecule includes a coding region sequence encoding the recombinant polypeptide and an IRES sequence operably linked to the coding region sequence. The source of the IRES sequence in the present disclosure includes, but is not limited to, Coxsackie virus, Echovirus, Taura syndrome virus, Triatoma virus, Theiler's encephalomyelitis virus, simian virus 40, Solenopsis invicta virus 1, Rhopalosiphum padi virus, reticuloendotheliosis virus, Forman poliomyelitis virus 1, Plautia stali intestine virus, Kashmir bee virus, human rhinovirus 2, Homalodisca coagulata virus-1, human immunodeficiency virus type 1, Himetobi P virus, hepatitis C virus, hepatitis A virus, hepatitis GB virus, foot-and-mouth disease virus, human enterovirus 71, equine rhinovirus, Ectropis obliqua-like virus, Encephalomyocarditis virus (EMCV), Drosophila C virus, Cruciferae tobacco virus, cricket paralysis virus, bovine viral diarrhea virus 1, black queen cell virus, aphid lethal paralysis virus, avian encephalomyelitis virus, acute bee paralysis virus, Hibiscus Chlorotic Ringspot virus, hog cholera virus, human FGF2, human SFTPA1, human AML1/RUNX1, Drosophila antenna, human AQP4, human AT1R, human BAG-1, human BCL2, human BiP, human c-IAPl, human c-myc, human eIF4G, mouse NDST4L, human LEF1, mouse HIF1α, human n.myc, mouse Gtx, human p27kipl, human PDGF2/c-sis, human p53, human Pim-1, mouse Rbm3, Drosophila reaper, dog Scamper, Drosophila Ubx, human UNR, mouse UtrA, human VEGF-A, human XIAP, Drosophila hairless, *Saccharomyces cerevisiae* TFIID, *Saccharomyces cerevisiae* YAP1, human c-src, human FGF-1, simian picornavirus, etc.

In some preferred embodiments, the IRES sequence includes the nucleotide sequence of any one or more in the group consisting of SEQ ID NOs: 14-20, or a sequence having at least 80%, 81%, 82%, 83%, 84%, 85%, 86%, 87%, 88%, 89%, 90%, 91%, 92%, 93%, 94%, 95%, 96%, 97%, 98%, 99% and 100% sequence identity with the nucleotide sequence in the group consisting of SEQ ID NOs: 14-20. In some embodiments, the IRES sequence is CVB3 IRES of the nucleotide sequence shown in SEQ ID NO: 14, EV24 IRES of the nucleotide sequence shown in SEQ ID NO: 15, EV29 IRES of the nucleotide sequence shown in SEQ ID NO: 16, and EV33 IRES of the nucleotide sequence shown in SEQ ID NO: 17. In some embodiments, the IRES sequence includes a chimera sequence of any one of CVB3v IRES and EV24 IRES, and EV29 IRES and EV33 IRES. In some embodiments, the IRES sequence includes a chimera sequence of CVB3v and EV24 IRES, and its nucleotide sequence is shown in SEQ ID NO: 18. In some embodiments, the IRES sequence includes a chimera sequence of CVB3v and EV29 IRES, and its nucleotide sequence is shown in SEQ ID NO: 19. In some embodiments, the IRES sequence includes a chimera sequence of CVB3v and EV33 IRES, and its nucleotide sequence is shown in SEQ ID NO: 20. The IRES sequence of the present disclosure can realize efficient and lasting expression of the recombinant polypeptide, and effectively improve the activity of the circular RNA molecule in targeted degradation of the protein of interest.

In some preferred embodiments, the circular RNA molecule further includes a second exon sequence located upstream of the IRES sequence, and the second exon (E2) sequence is a sequence having at least 80%, 81%, 82%, 83%, 84%, 85%, 86%, 87%, 88%, 89%, 90%, 91%, 92%, 93%, 94%, 95%, 96%, 97%, 98%, 99% and 100% sequence identity with the nucleotide sequence shown in SEQ ID NO: 22.

In some preferred embodiments, the circular RNA molecule further includes a first exon sequence located downstream of the coding region sequence, and the first exon (E1) sequence is a sequence having at least 80%, 81%, 82%, 83%, 84%, 85%, 86%, 87%, 88%, 89%, 90%, 91%, 92%, 93%, 94%, 95%, 96%, 97%, 98%, 99% and 100% sequence identity with the nucleotide sequence shown in SEQ ID NO: 21.

In some preferred embodiments, the circular RNA molecule includes a 5' spacer and a 3' spacer, wherein the 5' spacer sequence is located between the second exon E2 sequence and the IRES sequence, and the 3' spacer sequence is located between the first exon E1 sequence and the coding region sequence. Specifically, the 5' spacer sequence is a sequence having at least 80%, 81%, 82%, 83%, 84%, 85%, 86%, 87%, 88%, 89%, 90%, 91%, 92%, 93%, 94%, 95%, 96%, 97%, 98%, 99% and 100% sequence identity with the nucleotide sequence shown in any one of SEQ ID NOs: 23-24. The 3' spacer sequence is a sequence having at least 80%, 81%, 82%, 83%, 84%, 85%, 86%, 87%, 88%, 89%, 90%, 91%, 92%, 93%, 94%, 95%, 96%, 97%, 98%, 99% and 100% sequence identity with the nucleotide sequence shown in any one of SEQ ID NOs: 25-26. The 5' spacer and 3' spacer elements of a specific sequence in the circular RNA molecule can further prolong the time of expressing the polypeptide of interest in the coding region and improve the expression efficiency of the recombinant polypeptide.

In some specific embodiments, the circular RNA molecule includes the following sequences connected sequentially: the second exon sequence, the 5' spacer sequence, the IRES sequence, the coding region sequence, the 3' spacer sequence and the first exon sequence.

In some specific embodiments, the present disclosure provides a circular RNA molecule for targeted degradation of an eGFP protein, which encodes a recombinant polypeptide with the structure shown in (1). A nucleotide sequence of the circular RNA molecule is shown in SEQ ID NO: 2. Compared with a linear RNA molecule (with a nucleotide sequence as shown in SEQ ID NO: 2) for targeted degradation of the eGFP protein, the circular RNA molecule has a more significant protein degradation effect; and with the extension of time, the dominant effect of the circular RNA molecule in degrading an intracellular protein is more obvious.

In some specific embodiments, the present disclosure provides a circular RNA molecule for targeted degradation of a PCNA protein, which encodes a recombinant polypeptide with the structure shown in (2). A nucleotide sequence of the circular RNA molecule is shown in SEQ ID NO: 4. By comparing with a circular RNA molecule (with a nucleotide sequence as shown in SEQ ID NO: 5) encoding a recombinant polypeptide not having the E3 ubiquitin ligase activity, and comparing with a circular RNA molecule (with a nucleotide sequence as shown in SEQ ID NO: 6) encoding the recombinant polypeptide that cannot bind to the PCNA protein in a targeted manner, it is proved that the circular RNA molecule of the sequence shown in SEQ ID NO: 4 can effectively degrade the intracellular PCNA protein and has an excellent protein degradation effect.

In some specific embodiments, by injecting the circular RNA molecule of the sequence shown in SEQ ID NO: 2 into a living tumor tissue, it is found that the proportion of GFP-positive cells can be significantly reduced, indicating that the circular RNA molecule of the present disclosure is suitable for *in vivo* delivery and can play a protein degradation role *in vivo.* In some specific embodiments, by injecting the circular RNA molecule of the sequence shown in SEQ ID NO: 4 into the living tumor tissue, it is found that the tumor volume is reduced and the growth rate is inhibited, indicating that the circular RNA molecule of the present disclosure can exert an obvious therapeutic effect on the living tumor.

In some specific embodiments, the present disclosure provides a circular RNA molecule for targeted degradation of a KRAS protein, which encodes a recombinant polypeptide with the structure shown in (3). A nucleotide sequence of the circular RNA molecule is shown in SEQ ID NO: 46. By encoding a recombinant polypeptide that binds to the KRAS protein in a targeted manner and has the E3 ubiquitin ligase activity, the KRAS protein can be ubiquitinated by the enzyme activity of E3 ubiquitin ligase, which can realize efficient and specific degradation of the protein of interest.

In some specific embodiments, the present disclosure provides a circular RNA molecule for targeted degradation of a KRAS protein, which encodes a recombinant polypeptide with the structure shown in (4). A nucleotide sequence of the circular RNA molecule is shown in SEQ ID NO: 47. By encoding a recombinant polypeptide that binds to the KRAS protein in a targeted manner and has the E3 ubiquitin ligase activity, the KRAS protein can be ubiquitinated by the enzyme activity of E3 ubiquitin ligase, which can realize efficient and specific degradation of the protein of interest.

In some specific embodiments, the present disclosure provides a circular RNA molecule for targeted degradation of a KRAS protein, which encodes a recombinant polypeptide with the structure shown in (5). A nucleotide sequence of the circular RNA molecule is shown in SEQ ID NO: 48. By encoding a recombinant polypeptide that binds to the KRAS protein in a targeted manner and has the E3 ubiquitin ligase activity, the KRAS protein can be ubiquitinated by the enzyme activity of E3 ubiquitin ligase, which can realize efficient and specific degradation of the protein of interest.

In some specific embodiments, the present disclosure provides a circular RNA molecule for targeted degradation of a KRAS protein, which encodes a recombinant polypeptide with the structure shown in (6). A nucleotide sequence of the circular RNA molecule is shown in SEQ ID NO: 49. By encoding a recombinant polypeptide that binds to the KRAS protein in a targeted manner and has the E3 ubiquitin ligase activity, the KRAS protein can be ubiquitinated by the enzyme activity of E3 ubiquitin ligase, which can realize efficient and specific degradation of the protein of interest.

In some specific embodiments, the present disclosure provides a circular RNA molecule for targeted degradation of a KRAS protein, which encodes a recombinant polypeptide with the structure shown in (7). A nucleotide sequence of the circular RNA molecule is shown in SEQ ID NO: 50. By encoding a recombinant polypeptide that binds to the KRAS protein in a targeted manner and has the E3 ubiquitin ligase activity, the KRAS protein can be ubiquitinated by the enzyme activity of E3 ubiquitin ligase, which can realize efficient and specific degradation of the protein of interest.

In some specific embodiments, the present disclosure provides a circular RNA molecule for targeted degradation of a KRAS protein, which encodes a recombinant polypeptide with the structure shown in (8). A nucleotide sequence of the circular RNA molecule is shown in SEQ ID NO: 51. By encoding a recombinant polypeptide that binds to the KRAS protein in a targeted manner and has the E3 ubiquitin ligase activity, the KRAS protein can be ubiquitinated by the enzyme activity of E3 ubiquitin ligase, which can realize efficient and specific degradation of the protein of interest.

In some specific embodiments, the present disclosure provides a circular RNA molecule for targeted degradation of a KRAS protein, which encodes a recombinant polypeptide with the structure shown in (9). A nucleotide sequence of the circular RNA molecule is shown in SEQ ID NO: 52. By encoding a recombinant polypeptide that binds to the KRAS protein in a targeted manner and has the E3 ubiquitin ligase activity, the KRAS protein can be ubiquitinated by the enzyme activity of E3 ubiquitin ligase, which can realize efficient and specific degradation of the protein of interest.

In some specific embodiments, the present disclosure provides a circular RNA molecule for targeted degradation of a KRAS protein, which encodes a recombinant polypeptide with the structure shown in (10). A nucleotide sequence of the circular RNA molecule is shown in SEQ ID NO: 53. By encoding a recombinant polypeptide that binds to the KRAS protein in a targeted manner and has the E3 ubiquitin ligase activity, the KRAS protein can be ubiquitinated by the enzyme activity of E3 ubiquitin ligase, which can realize efficient and specific degradation of the protein of interest.

In some specific embodiments, the present disclosure provides a circular RNA molecule for targeted degradation of a KRAS protein, which encodes a recombinant polypeptide with the structure shown in (11). A nucleotide sequence of the circular RNA molecule is shown in SEQ ID NO: 54. By encoding a recombinant polypeptide that binds to the KRAS protein in a targeted manner and has the E3 ubiquitin ligase activity, the KRAS protein can be ubiquitinated by the enzyme activity of E3 ubiquitin ligase, which can realize efficient and specific degradation of the protein of interest.

In some specific embodiments, the present disclosure provides a circular RNA molecule for targeted degradation of a KRAS protein, which encodes a recombinant polypeptide with the structure shown in (12). A nucleotide sequence of the circular RNA molecule is shown in SEQ ID NO: 55. By encoding a recombinant polypeptide that binds to the KRAS protein in a targeted manner and has the E3 ubiquitin ligase activity, the KRAS protein can be ubiquitinated by the enzyme activity of E3 ubiquitin ligase, which can realize efficient and specific degradation of the protein of interest.

In some embodiments, the present disclosure provides a cyclization precursor RNA molecule that can be cyclized to form the aforementioned circular RNA molecule.

In some preferred embodiments, the cyclization precursor RNA molecule further includes a 5' homologous arm sequence located upstream of the second exon sequence, and the 5' homologous arm sequence is a sequence having at least 80%, 81%, 82%, 83%, 84%, 85%, 86%, 87%, 88%, 89%, 90%, 91%, 92%, 93%, 94%, 95%, 96%, 97%, 98%, 99% and 100% sequence identity with the nucleotide sequence shown in any one of SEQ ID NOs: 29-30.

In some preferred embodiments, the cyclization precursor RNA molecule further includes a 3' homologous arm sequence located downstream of the first exon sequence. The 3' homologous arm sequence is a sequence having at least 80%, 81%, 82%, 83%, 84%, 85%, 86%, 87%, 88%, 89%, 90%, 91%, 92%, 93%, 94%, 95%, 96%, 97%, 98%, 99% and 100% sequence identity with the nucleotide sequence shown in any one of SEQ ID NOs: 31-32.

In some preferred embodiments, the cyclization precursor RNA molecule further includes a 3' intron sequence located between the second exon sequence and the 5' homologous arm sequence, and the 3' intron sequence is a sequence having at least 80%, 81%, 82%, 83%, 84%, 85%, 86%, 87%, 88%, 89%, 90%, 91%, 92%, 93%, 94%, 95%, 96%, 97%, 98%, 99% and 100% sequence identity with the nucleotide sequence shown in SEQ ID NO: 28.

In some preferred embodiments, the cyclization precursor RNA molecule further includes a 5' intron sequence located between the first exon sequence and the 3' homologous arm sequence, and the 5' intron sequence is a sequence having at least 80%, 81%, 82%, 83%, 84%, 85%, 86%, 87%, 88%, 89%, 90%, 91%, 92%, 93%, 94%, 95%, 96%, 97%, 98%, 99% and 100% sequence identity with the nucleotide sequence shown in SEQ ID NO: 27.

In some specific embodiments, the cyclization precursor RNA molecule includes the following sequences connected sequentially:
a 5' homologous arm sequence, a 3' intron sequence, a second exon sequence, a 5' spacer sequence, an IRES sequence, a coding region sequence, a 3' spacer sequence, a first exon sequence, a 5' intron sequence and a 3' homologous arm sequence.

The cyclization precursor RNA molecule is cyclized through the following process: by means of the ribozyme characteristics of the intron, the connection between the 5' intron and the first exon is broken under the trigger of GTP; the cleavage site of ribozyme in the first exon further attacks the connection between the 3' intron and the second exon and causes the connection to break, so that the 3' intron is dissociated, and the first exon and the second exon are connected to form circular RNA.

In some embodiments, the present disclosure provides a recombinant nucleic acid molecule that can be transcribed to form the aforementioned cyclization precursor RNA molecule. In order to further transcribe the recombinant nucleic acid molecule into an RNA molecule, a regulatory sequence may also be included in the recombinant nucleic acid molecule. Exemplarily, the regulatory sequence is a T7 promoter linked to the upstream of the 5' homologous arm.

In some embodiments, the present disclosure provides a recombinant expression vector, including the aforementioned recombinant nucleic acid molecule. The vector for connecting the recombinant nucleic acid molecule can be various vectors commonly used in the art, such as a pUC57 plasmid, etc. Further, the recombinant nucleic acid molecule contains a restriction enzyme cleavage site, so that the recombinant expression vector is digested to obtain a linearized vector suitable for transcription.

### Composition

The composition provided by the present disclosure includes the circular RNA according to the present disclosure and has excellent activity of targeted degradation of protein. The composition is suitable for delivery in to the body, which can solve the problem that the existing Bio-PROTAC molecule cannot achieve targeted degradation of the protein of interest in the body.

In some embodiments, the composition further includes one or more pharmaceutically acceptable carriers. In the present disclosure, the purpose of using the composition is to promote the administration to an organism, facilitate the absorption of active ingredients, and in turn exert biological activity. The composition of the present invention can be administered in any form, including injection (intra-arterial, intravenous, intramuscular, intraperitoneal, subcutaneous), mucosal, oral (oral solid preparation, oral liquid preparation), rectal, inhalation, implantation, topical (e.g. ocular) administration, etc. Non-limiting examples of the oral solid preparation include, but are not limited to, powder, capsules, lozenges, granules, tablets, etc. Non-limiting examples of the liquid preparation for oral or mucosal administration include, but are not limited to, suspensions, tinctures, elixirs, solutions, etc. Non-limiting examples of the preparation for topical administration include, but are not limited to, emulsions, gels, ointments, creams, patches, pastes, foams, lotions, drops or serum preparations. Non-limiting examples of the preparation for parenteral administration include, but are not limited to, solutions for injection, dry powder for injection, suspensions for injection, emulsions for injection, etc. The composition of the present invention can also be made into controlled-release or delayed-release dosage forms (e.g., liposomes or microspheres).

In the present disclosure, the route of administration can be changed or adjusted in any suitable way to meet requirements of the drug nature, the convenience for patients and medical staff and other related factors.

In some preferred embodiments, the composition only consists of a circular RNA molecule and a solvent for dissolving the circular RNA molecule. Exemplarily, the solvent is a 1×TE buffer or any other type of solvent capable of dissolving the circular RNA molecule. The present disclosure has unexpectedly found that, the circular RNA molecule in the present disclosure does not need to be wrapped with lipid nanoparticles (LNPs) and the like, i.e., having high stability, can give full play to the activity of targeted degradation of the protein of interest after being delivered into the body, and can realize the specific and efficient degradation of a disease-associated protein, thereby achieving the effect of disease treatment.

### Use of circular RNA or composition

In some embodiments, the present disclosure provides use of the circular RNA molecule or the composition including the circular RNA molecule in targeted degradation of a protein of interest. For the first time, the present disclosure realizes the delivery of the circular RNA molecule into cells and the targeted degradation of the protein of interest. Compared with the small molecular proteolysis-targeting chimera or the macromolecular Bio-PROTACs, the circular RNA molecule in the present disclosure is easily delivered into the cells, which can achieve high-efficiency degradation of the protein of interest in the cells. Compared with linear mRNA, the circular RNA molecule in the present disclosure has a better protein translation level and expression persistence, and causes a more remarkable protein degradation effect.

In some preferred embodiments, the present disclosure provides use of the circular RNA molecule or the composition including the circular RNA molecule in targeted degradation of a protein of interest in a subject. The present disclosure has unexpectedly found that, the circular RNA molecule effectively inhibits the growth of the tumor after being administrated to a living tumor of a subject, which proves that the circular RNA molecule has excellent activity of *in vivo* targeted degradation of protein, which completely solves the problem that the Bio-PROTACs cannot achieve *in vivo* protein degradation.

In some embodiments, the present disclosure provides use of the circular RNA molecule or the composition including the circular RNA molecule in reducing or eliminating the expression of a protein of interest. The circular RNA molecule in the present disclosure has significantly improved intracellular protein degradation activity, and is suitable for acting as or preparing an agent for targeted degradation of the protein of interest, especially acting as or preparing an agent for targeted degradation of the protein of interest in a subject.

In some embodiments, the present disclosure provides use of the circular RNA molecule or the composition including the circular RNA molecule in acting as or preparing a drug for treating a disease. The circular RNA molecule can achieve specific and efficient degradation of the disease-associated protein after being administrated into the subject, so as to achieve the effect of disease treatment.

In some preferred embodiments, the present disclosure provides use of the circular RNA molecule or the composition including the circular RNA molecule in acting as or preparing a drug for treating tumor. For the first time, a nude mouse tumor model is injected with the circular RNA molecule through intratumor injection in the present disclosure, which successfully realizes inhibition of tumor growth, and proves for the first time that the circular RNA molecule can be applied for tumor treatment, which has an important medical application prospect.

### Examples

Other objectives, features and advantages of the present disclosure will become apparent from the following detailed description. However, it should be understood that the detailed description and specific examples (although indicating the specific embodiments of the present disclosure) are given for explanatory purposes only, because various changes and modifications made within the spirit and scope of the present disclosure will become apparent to those skilled in the art after reading the detailed description.

The experimental techniques and experimental methods used in the examples are conventional technical methods, unless otherwise specified. For example, the experimental methods in the following examples which are not specified with specific conditions are generally carried out according to conventional conditions, such as those described in Sambrook et al., Molecular Cloning: Laboratory Manual (New York: Cold Spring Harbor Laboratory Press, 1989), or those recommended by the manufacturer. The materials, reagents, etc. used in the examples are all normally commercially available, unless otherwise specified.

### Example 1: Verification of degradation effect of eGFP protein mediated by eFGP-targeting degrader fusion protein encoded by circular RNA in 293T cells

### 1.1 Experimental method and steps:

(1) construction of plasmid such as EV29-H2B-eGFP
A plasmid expressing the gene of interest H2B-eGFP and a plasmid expressing degraders of H2B-eGFP were constructed. This step was entrusted to Suzhou Genwitz Biotechnology Co., Ltd. for gene synthesis and cloning. A DNA vector used here for constructing circular RNA included a T7 promoter, a 5' homologous arm, a 3' intron, a second exon E2, a 5' spacer, an IRES element, a coding region of H2B-eGFP and an eGFP-targeting degrader fusion protein, a downstream spacer, a 5' intron, a first exon E1, a 3' homologous arm, and a cleavage site XbaI which could be used for plasmid linearization. A gene fragment obtained for linear mRNA TRNP-eGFP-DASP was ligated to a pUC57 vector.

**Table 1**

| Name of protein sequence | Name of RNA sequence | Name of plasmid | RNA sequence |
|---|---|---|---|
| H2B-eGFP | EV29-H2B-eGFP | pUC57-EV29-H2B-eGFP | SEQ ID No: 1 |
| eGFP-DASP | EV29-eGFP-DASP | pUC57-EV29-eGFP-DASP | SEQ ID No: 2 |
| eGFP-DASP | TRNP-eGFP-DASP | pUC57-TRNP-eGFP-DASP | SEQ ID No: 3 |

(2) preparation of linear plasmid template for *in vitro* transcription such as EV29-H2B-eGFP
1) plasmid extraction
   1. externally synthesized puncture bacteria were activated at 37°C and 220 rpm for 3-4 h;
   2. a solution of the activated bacteria was taken for expanded culture under the culture conditions of 37°C/220 rpm/overnight;
   3. plasmid extraction (Tiangen endotoxin-free small amount Midiprep kit) was conducted, and an OD value was determined.
2) enzyme digestion of plasmid

An Xba I single enzyme digestion method was adopted to digest the aforementioned plasmid prepared in 1).

An enzyme digestion system was as follows:

**Table 2**

| Agent | Volume |
|---|---|
| Plasmid | 10 µg |
| Enzyme (1000 units) | 5 µl |
| 10× cutsmart buffer | 30 µl |
| Nuclease free, H₂O | Total 300 µl |

Enzymatic digestion was conducted at 37°C overnight: the enzymatic digestion products were recovered by a method of directly passing through a column (Tiangen universal DNA gel recovery kit), the OD value was determined, and the products were identified by 1% agarose gel electrophoresis; and the purified linear plasmid template was used for transcription *in vitro.*
(3) synthesis of circular mRNA
1) synthesis of mRNA by transcription *in vitro*
a transcription system was as follows:

**Table 3**

| Agent | Volume |
|---|---|
| 10× Reaction Buffer | 2 µl |
| ATP (20 mM) | 2 µl |
| CTP (20 mM) | 2 µl |
| UTP (20 mM) | 2 µl |
| GTP (20 mM) | 2 µl |
| linearized DNA from Xba I | X µl (500-1000 ng) |
| single enzyme digestion | |
| T7 RNA Polymerase | 2 µl |
| RNA inhibitor | 2 µl |
| RNA Nuclease free, H₂O | Total 20 µl |

Incubation was carried out at 37°C for 2-3 h, then 20 µl of the transcription system was digested with 2 µl of DNaseI under the digestion conditions of 37°C for 15 min to remove the linear DNA template.
2) mRNA purification
The transcription product obtained in 1) was purified by a silicon film centrifugal column (Thermo, GeneJET RNA Purification Kit), the OD value was determined, and the RNA size was identified by 1% denatured agarose gel electrophoresis.
3) mRNA cyclization:
Cyclization agent: GTP buffer: 50 mM Tris-HCl, 10 mM MgCl₂, 1 mM DTT, pH of about 7.5

Cyclization system and conditions:

**Table 4**

| Solution | Volume |
|---|---|
| mRNA | 25 µg RNA solution |
| GTP solution (20 mM) | 50 µl |
| GTP buffer | made up to 500 µl |

The aforementioned solution was heated at 55°C for 15 min, the cyclized RNA product was purified by a silicon film centrifugal column (Thermo, GeneJET RNA Purification Kit), and the OD value was determined.

### (4) Synthesis of linear mRNA

The methods of linearization by digestion and template purification of the pUC57-TRNP-eGFP-DASP plasmid were the same as those in (2)-2). Linear mRNA having a Cap1 structure was synthesized by transcription *in vitro* with an APExBio kit HyperScribe^{™} All in One mRNA Synthesis Kit II, and then subjected to nucleoside modification with pseudouridine. Finally, the linear mRNA having the Cap1 structure, a PolyA tail and the pseudouridine modification was obtained.

### (5) Cell culture and transfection:

1) cell culture:
The 293T was inoculated into a DMEM high-sugar medium containing 10% of fetal bovine serum and 1% of penicillin and streptomycin solution, and cultured in a 5% CO₂ incubator at 37°C. Cells were subcultured every other 2-3 days.
2) cell transfection:
Before transfection, the 293T cells were seeded into a 24-well plate at 1 × 10⁵ cells/well, and cultured in a 5% CO₂ incubator at 37°C. After the cells reached a confluence of 70-90%, a transfection reagent Lipofectamine MessengerMax (Invitrogen) was used for transfecting the EV29-H2B-eGFP mRNAs with different masses firstly, and then after 6 hours, for transfecting the EV29-eGFP-DASP mRNA or eGFP-DASP linear mRNA into 293T cells at different proportions. The specific operations were as follows:
1. Dilution of an MessengerMAX^{™} Reagent

**Table 5**

| Agent | Volume/well |
|---|---|
| MEM serum-free medium | 25 µl |
| Messenger MAX^{™} Reagent | 0.75 µl |

After dilution and mixing, incubation was carried out by standing at room temperature for 10 min.
2. Dilution of mRNA

**Table 6**

| Agent | Volume/well |
|---|---|
| mRNA | Calculated according to the actual transfection amount |
| MEM serum-free medium | made up to 25 µl |

3. The MessengerMAX^{™} Reagent and mRNA (1:1) were taken after mixing and diluting

**Table 7**

| Reagent | Volume/well |
|---|---|
| Diluted Messenger MAX^{™} Reagent | 25 µl |
| Diluted mRNA | 25 µl |

After dilution and mixing, incubation was carried out by standing at room temperature for 5min.
4. 50 µl of the aforementioned mixed solution was sucked and slowly added into a 24-well plate in an adherent manner, and incubation was carried out in a 5% CO₂ incubator at 37°C.
3) Detection of eGFP protein expression
1. Observation of cell fluorescence: the expression of eGFP in the 293T cells 24 hours and 48 hours after transfection was observed under a 200X fluorescence microscope.
2. Detection of the median fluorescence intensity of cells by flow cytometry: the median fluorescence intensity of the 293T cells was detected by a flow cytometer 24 h and 48 h after transfection.

### 1.2 Experimental results

1) preparation of DNA transcription template
   1. The plasmid extraction concentration was: pUC57-EV29-H2B-eGFP: 557.8 ng/µl, and pUC57-EV29-eGFP-DASP: 565.2 ng/µl;
   2. The concentration of plasmid digestion and linearization: pUC57-EV29-H2B-eGFP: 148 ng/µl, and pUC57-EV29-eGFP-DASP: 142.3 ng/µl;
2) mRNA transcription and cyclization
   1. Concentration of transcribed and purified mRNA: EV29-H2B-eGFP: 914.4 ng/µl, and EV29-eGFP-DASP: 491.8 ng/µl;
   2. The concentration of mRNA after cyclization and purification: EV29-H2B-eGFP: 201 ng/µl, and EV29-eGFP-DASP: 108 ng/µl;
   3. 97 ng/µl of the linear mRNA having the Cap1 structure of eGFP-DASP and pseudouridine modified nucleosides was obtained after *in vitro* transcription and purification.
3) detection of protein expression

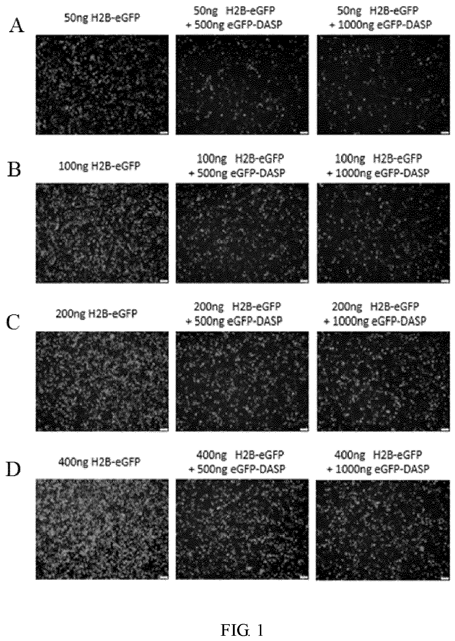

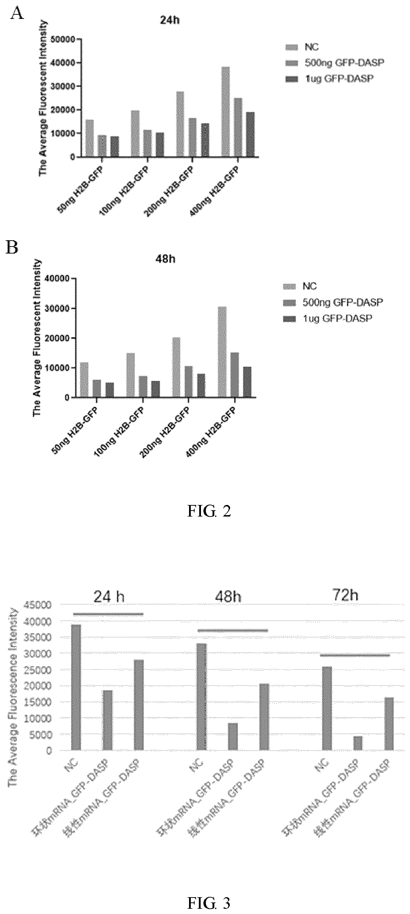

As shown in FIG. 1 (fluorescence of H2B-eGFP 48 hours after transfection) and FIG. 2 (flow cytometry quantification): the fluorescence observation and flow cytometry data showed that when 50 ng, 100 ng, 200 ng and 400 ng of the EV29-H2B-eGFP mRNA were transfected initially, the fluorescence intensity of the expressed eGFP protein was increased with the increase of transfection amount. On this basis, after the transfection of the EV29-eGFP-DASP mRNA, the fluorescence intensity of the eGFP protein was decreased significantly, and the fluorescence intensity was decreased with the increase of the amount of the transfected EV29-eGFP-DASP mRNA. It was indicated that the protein translated from EV29-eGFP-DASP can have a degradation effect on the H2B-eGFP protein. As shown in FIG. 3, by comparing the protein degradation effects of the linear mRNA and the circular mRNA encoding the same Bio-PROTACs with the same quantity (with the same transfection quantity of 1 µg) on the H2B-eGFP (with the quantity of the transfected mRNA of 400 ng), it was found that the protein degradation effect of the circular mRNA on the H2B-eGFP was significantly better than that of the linear mRNA, and with the extension of time (up to 72 hours), the protein degradation advantage of the circular mRNA was further increased.

### Example 2: Verification of degradation effect of PCNA protein mediated by PCNA-targeting degrader fusion protein encoded by circular RNA in A549 cells

### 2.1 Experimental method and steps

(1) construction of plasmid such as EV29-CON1-SP-WT
On the basis of the aforementioned Example 1, the same circular RNA element as that of the aforementioned Example 1 was adopted to construct a gene sequence EV29-CON1-SP-WT that encodes a PCNA protein degradation-targeting fusion protein, and gene sequences pUC57-EV29-CON1-SP-mu and pUC57-EV29-CON1-mu-SP that encode fusion proteins with loss of function. The information was shown below. DNA synthesis was entrusted to Suzhou Genwitz Biotechnology Co., Ltd.

**Table 8**

| Name of protein sequence | Name of RNA sequence | Name of plasmid | RNA SEQ.ID |
|---|---|---|---|
| CON1-SP-WT | EV29-CON1-SP-WT | pUC57-EV29-CON1-SP-WT | SEQ.ID No: 4 |
| CON1-SP-mu | EV29-CON1-SP-mu | pUC57-EV29-CON1-SP-mu | SEQ.ID No: 5 |
| CON1-mu-SP | EV29-CON1-mu-SP | pUC57-EV29-CON1-mu-SP | SEQ.ID No: 6 |

(2) methods of the linearization of the plasmid DNA, *in vitro* transcription of the cyclization precursor mRNA, purification of the cyclization precursor mRNA, cyclization reaction of mRNA, purification of the circular mRNA, cell culture and transfection, and the like are all the same as those in 1.1 of Example 1, and the cell type was changed to a tumor cell line A549 for expressing PCNA.
(3) detection of cellular PCNA protein level by Western Blot

### 1) preparation of SDS-PAGE gel

The gel with the desired concentration was formulated according to the table below:

**Table 9**

| Name of component | the dosage of each component corresponding to each gel volume (ml) | | | |
|---|---|---|---|---|
| | 5 ml | 10ml | 15ml | 20ml |
| H₂O | 1.9 | 4.0 | 5.9 | 7.9 |
| 30% Acrylamide | 1.7 | 3.3 | 5.0 | 6.7 |
| 1.5M Tris-HCl (pH 8.8) | 1.3 | 2.5 | 3.8 | 5.0 |
| 10% SDS | 0.05 | 0.1 | 0.15 | 0.2 |
| 10% ammonium persulfate | 0.05 | 0.1 | 0.15 | 0.2 |
| TEMED | 0.002 | 0.004 | 0.006 | 0.008 |

After TEMED was added, uniform mixing was immediately performed, the obtained mixture was slowly added into a gel bed between two glass plates, and then a layer of water was added on the gel; after separation gel in a lower layer was solidified (a refraction line could be seen between water and the gel), a water layer in an upper layer was poured out, and a required volume of concentrated gel in the upper layer was formulated according to the table below:

**Table 10**

| Name of component | the dosage of each component corresponding to each gel volume (ml) | | | |
|---|---|---|---|---|
| | 2ml | 4ml | 5 ml | 6ml |
| H₂O | 1.4 | 2.7 | 3.4 | 4.1 |
| 30% Acrylamide | 0.33 | 0.67 | 0.83 | 1.0 |
| 1.5M Tris-HCl (pH 8.8) | 0.25 | 0.5 | 0.63 | 0.75 |
| 10% SDS | 0.02 | 0.04 | 0.05 | 0.06 |
| 10% ammonium persulfate | 0.02 | 0.04 | 0.05 | 0.06 |
| TEMED | 0.002 | 0.004 | 0.005 | 0.006 |

After TEMED was added, uniform mixing was immediately performed. The concentrated gel was slowly added to fill up the remaining space, then a comb was inserted into the concentrated gel, and electrophoresis could be carried out after the concentrated glue in the upper layer was solidified.

### 1) protein sample loading and electrophoresis

A protein sample was subjected to denaturing treatment before loading: 5×SDS Loading was added to the protein sample until the final concentration was 1×, full mixing was performed, and then the mixture was heated at 95°C for 5 min; and electrophoresis was conducted after sample loading: electrophoresis was conducted at a constant voltage of 100 V for 20 min, then the voltage was changed to 120 V, and electrophoresis was conducted. The electrophoresis could be terminated after a target band was fully separated, and then transfer was conducted.

### 2) Transfer

A PVDF film with an appropriate size was cut according to the protein sample loading and detection range, and soaked in a methanol solution for several minutes; a gel plate after electrophoresis was taken out, excess gel was peeled off, and then the gel plate was put on filter paper, and put into a transfer tank in the order of clamp plate negative electrode-sponge pad-filter paper-gel-PVDF film-filter paper-sponge pad-clamp plate positive electrode. Transfer was conducted at a constant current of 200 mA for 1.5 hours, and since heat was generated during electric transfer, it is necessary to place ice cubes around the tank to cool down.

### 3) immune reaction

After transfer was completed, the film was transferred into a flat dish containing a blocking liquid (the blocking liquid is 5% milk formulated in TBST), and blocked with shaking in a shaker at room temperature for 1 hour; a primary antibody was diluted with 5% milk to an appropriate working concentration, added into the flat dish, preferably to cover the film, and incubated in the shaker at room temperature for 1-2 h or at 4°C overnight; washing was performed with TBST on a decolorizing shaker at room temperature for three times, each time for 10 min; the TBST washing solution was discarded, a secondary antibody was diluted with 5% milk to an appropriate working concentration, added into the flat dish, preferably to cover the film, and incubated in the shaker at room temperature for 1-2 h; and washing was performed with TBST on the decolorizing shaker at room temperature for three times, each time for 10 min;

### 4) Chemiluminescence development

Two reagents A and B of ECL were mixed in equal volume in an EP tube, then evenly added dropwise on the protein surface of the PVDF film, reacted for 1-2 min and then developed;

### 2.2 Experimental results

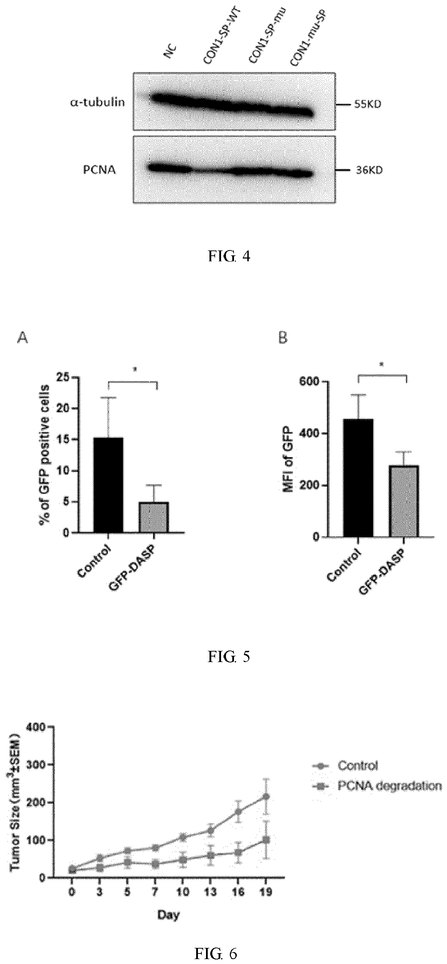

1) preparation of DNA linearization template
   1. The extraction concentration of plasmid: pUC57-EV29-CON1-SP-WT: 404.4 ng/µl, pUC57-EV29-CON1-SP-mu: 775.8 ng/µl, and pUC57-EV29-CON1-mu-SP: 563.4 ng/µl;
   2. The concentration of plasmid digestion and linearization: pUC57-EV29-CON1-SP-WT: 144.4 ng/µl, pUC57-EV29-CON1-SP-mu: 157.3 ng/µl, and pUC57-EV29-CON1-mu-SP: 149.8 ng/µl;
2) mRNA transcription and cyclization
   1. Concentration of transcribed and purified mRNA: EV29-CON1-SP-WT: 1788.8 ng/µl, EV29-CON1-SP-mu: 1716.8 ng/µl, and EV29-CON1-mu-SP: 1347.4 ng/µl;
   2. The concentration of mRNA after cyclization and purification: EV29-CON1-SP-WT: 2097.4 ng/µl, EV29-CON1-SP-mu: 2515.5 ng/µl, and EV29-CON1-mu-SP: 2160 ng/µl;
3) The expression level of the PCNA protein was detected 24 hours after cell transfection: the results of Western Blot showed (FIG. 4) that compared with a blank control group (without transfection of circular RNA) and a negative control group (transfection of a circular RNA encoding loss of the degradation function), the expression level of the PCNA protein in cells of the group transfected with EV29-CON1-SP-WT mRNA was lower, indicating that EV29-CON1-SP-WT mRNA could realize the degradation effect on the PCNA protein in the A549 cells.

### Example 3: Verification of in vivo degradation effect of eGFP protein mediated by eGFP-targeting degrader fusion protein encoded by circular mRNA

### 3.1 Experimental method

1) methods of the linearization of the plasmid DNA, *in vitro* transcription of the cyclization precursor mRNA, purification of the cyclization precursor mRNA, cyclization reaction of mRNA, purification of the circular mRNA, cell culture, and the like are all the same as those in 1.1 of Example 1, and the cell type was changed to a tumor cell line A549.
2) cell collection: the cells were digested with 0.25% trypsin-EDTA (Gibco), and centrifuged at 1,000 rpm for 5 min, and the resulting supernatant was discarded. The cells were resuspended in a phosphate buffer in which Matrigel (Corning) was added, and then counted.
3) A549 tumor model: female CD-1 Nude mice (6-7 weeks old) were subcutaneously inoculated with 3 × 10⁶ A549 cells, and a tumor size was measured with a vernier caliper twice a week until the end of the experiment. When the tumor size reached about 2,000 mm³ or there was an animal health problem (with ulcer occurred on 20% area of the tumor), the animal was euthanized. The data was sorted out by using GraphPad Prism 8.0.2, and a tumor growth curve graph was plotted.
4) injection of circular RNA: after two weeks of modeling, each mouse was injected with 20 µg of the circular RNA of EV29-H2B-eGFP intratumorally, and the nude mice were randomly divided into two groups. 6 hours later, one group was injected with 20 µg of a circular RNA (EV29-eGFP-DASP) encoding GFP degradation intratumorally, and the other group was injected with 20 µg of control mRNA (control). 40 hours later, the nude mice were euthanized and the tumor was stripped off.
5) Single cell suspension: the tumor tissues were put in a 24-well plate containing a DMEM basic medium, and the 24-well plate was placed onto ice. The DMEM basic medium was replaced with 5 mL of a DMEM basic medium containing 1 mg/mL of collagenase IV (Thermo Fisher Scientific) and 100 U/mL of DnaseI, and the tumor was cut into small pieces of about 1 mm³ with scissors. The tumor pieces were transferred into a 15 mL centrifuge tube together with a digestive fluid, and incubated in a shaker at 37°C, at a rotation speed of 70 r/min for 30 minutes. After completion of digestion, 5 mL of a DMEM complete medium containing 10% inactivated fetal bovine serum was added to the centrifuge tube to stop digestion, and then centrifugation was performed after passing through a cell screen of 70 µm.
6) cell fluorescence detection: a solution of Fixable Cell Viability dye efluor 780 (Thermo Fisher Scientific) in PBS was added to the prepared single cell suspension, incubation was carried out at 4°C with protection from light for 20 minutes, centrifugation was performed, washing was performed once with a PBS solution, the cells were resuspended with 200 µl of the PBS solution, and dead cells were stained. The proportion of GFP positive cells in each group of tumor cells and the GFP median fluorescence intensity of the total cells were detected and analyzed by a flow cytometer.

### 3.2 Experimental results

As shown in FIG. 5, intratumoral injection of the circular RNA (EV29-eGFP-DASP) for degradation of GFP could significantly reduce the proportion of GFP-positive cells in the tumors and the GFP median fluorescence intensity. This result intuitively showed that the circular RNA could degrade the protein of interest *in vivo.*

### Example 4: Verification of in vivo degradation effect of PCNA protein mediated by PCNA-targeting degrader fusion protein encoded by circular mRNA

### 4.1 Experimental method

1) methods of the linearization of the plasmid DNA, *in vitro* transcription of the cyclization precursor mRNA, purification of the cyclization precursor mRNA, cyclization reaction of mRNA, purification of the circular mRNA, cell culture, and the like are all the same as those in 2.1 of Example 2.
2) methods of the cell collection, tumor modeling and the like were the same as those in 3.1 of Example 3.
3) When the tumors grown to about 2 mm × 3 mm, the nude mice were randomly divided into a control group and a PCNA degradation group, where n was equal to 5 in the control group, and n was equal to 6 in the PCNA degradation group. The control group was the circular RNA EV29-CON1-SP-mu with loss of the degradation function, and the experimental group was the circular RNA EV29-CON1-SP-WT with the function of degrading PCNA. Every other 2 days, the mice were injected with 10 µg of the control RNA or the RNA with the function of degrading PCNA intratumorally. While administration, a tumor volume was measured, and a tumor growth curve was plotted. At the end of the experiment, the nude mice were euthanized, and the tumors were stripped off and photographed.

### 4.2 Experimental results

As shown in FIG. 6, since the tumor model adopted a nude mouse model and a human non-small cell lung cancer cell line, while the EV29-CON1-SP-WT mRNA targets a human PCNA protein, after injection with this set of RNAs, the growth rate of the tumor volume in the mice was gradually slowed down, and the tumor size was significantly smaller than that in the group injected with the negative control RNA. This experiment verified that the circular RNA could degrade the protein of interest *in vivo*, and thereby the function of the protein of interest was affected.

### Example 5: Verification of degradation effect of KRAS protein mediated by KRAS-targeting degrader fusion protein encoded by circular RNA in H358 cells

### Experimental method and steps: construction of plasmids such as EV29-K19-VHK/VHKC

On the basis of the aforementioned Example 1, the same circular RNA element as that of the aforementioned Example 1 was adopted to construct a gene sequence EV29-K19-VHK that encodes a KRAS protein degradation-targeting fusion protein, and a gene sequence EV29-K19-VHKC that encodes a fusion protein without the VHL function of E3 ligase. The information was shown below. DNA synthesis was entrusted to Suzhou Genwitz Biotechnology Co., Ltd.

**Table 11**

| Name of protein sequence | Name of RNA sequence | Name of plasmid | RNA SEQ.ID |
|---|---|---|---|
| K19-VHK | EV29-K19-VHK | pUC57-EV29-K19-VHK | SEQ.ID No: 55 |
| K19-VHKC | EV29-K19-VHKC | pUC57-EV29-K19-VHKC | SEQ.ID No: 56 |

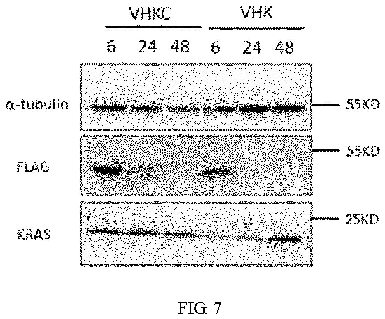

Methods of the linearization of the plasmid DNA, in vitro transcription of the cyclization precursor mRNA, purification of the cyclization precursor mRNA, cyclization reaction of mRNA, purification of the circular mRNA, cell culture and transfection, and the like are all the same as those in 1.1 of Example 1, and the cell type was changed to a tumor cell line H358 for expressing KRAS. The experimental steps of cell transfection and Western Blot were the same as those in Example 2. The KRAS protein level of the H358 cells was tested by Western Blot at 6 h, 24 h and 48 h after the cell transfection, respectively. As shown in FIG. 7, the level of the KRAS protein in the H358 cells was decreased significantly 6 and 24 hours after transfection of the circular RNA EV29-VHK, while the KRAS level in the H358 cells was not changed significantly after transfection of the circular RNA EV29-VHKC, indicating that the circular RNA EV29-VHK led to significant degradation of the KRAS protein.

### Example 6: Verification of degradation effect of KRAS protein mediated by KRAS-targeting degrader fusion protein constructed by different E3 ligases and encoded by circular RNA in H358 cells

Experimental method and steps:
On the basis of the aforementioned Example 1, the same circular RNA element as that in the aforementioned Example 1 was adopted to construct gene sequences EV29-βTrCP-K19, EV29-FBW7-K19, EV29-SKP2-K19, EV29-CRBN-K19, EV29-DDB2-K19, EV29-K19-SOCS2, EV29-K19-ASB1, EV29-K19-CHIP, and EV29-K19-SPOP that encode KRAS protein degradation-targeting fusion proteins.

The information was shown in the table below. DNA synthesis was entrusted to Suzhou Genwitz Biotechnology Co., Ltd.

**Table 12**

| Name of protein sequence | Name of RNA sequence | Name of plasmid | RNA SEQ.ID |
|---|---|---|---|
| BTrCP-K19 | EV29-βTrCP-K19 | pUC57-EV29-βTrCP-K19 | SEQ.ID No: 36 |
| FBW7-K19 | EV29-FBW7-K19 | pUC57-EV29-FBW7-K19 | SEQ.ID No: 37 |
| SKP2-K19 | EV29-SKP2-K19 | pUC57-EV29-SKP2-K19 | SEQ.ID No: 38 |
| CRBN-K19 | EV29-CRBN-K19 | pUC57-EV29-CRBN-K19 | SEQ.ID No: 39 |
| DDB2-K19 | EV29-DDB2-K19 | pUC57-EV29-DDB2-K19 | SEQ.ID No: 40 |
| K19-SOCS2 | EV29-K19-SOCS2 | pUC57-EV29-K19-SOCS2 | SEQ.ID No: 41 |
| K19-ASB1 | EV29-K19-ASB1 | pUC57-EV29-K19-ASB1 | SEQ.ID No: 42 |
| K19-CHIP | EV29-K19-CHIP | pUC57-EV29-K19-CHIP | SEQ.ID No: 43 |
| K19-SPOP | EV29-K19-SPOP | pUC57-EV29-K19-SPOP | SEQ.ID No: 44 |

Methods of the linearization of the plasmid DNA, in vitro transcription of the cyclization precursor mRNA, purification of the cyclization precursor mRNA, cyclization reaction of mRNA, purification of the circular mRNA, cell culture and transfection, and the like are all the same as those in 1.1 of Example 1, and the cell type was changed to a tumor cell line H358 for expressing KRAS. The experimental steps of cell transfection and Western Blot were the same as those in Example 2. The KRAS protein level of the H358 cells was tested by Western Blot at 6 hours after the cell transfection. The degradation effect on the KRAS protein was shown in Table 13. By taking the protein level of EV29-K19-VHKC as a control, the percentages of the KRAS protein level after transfection of different circular RNAs were calculated. The experimental results showed that adoption of the fusion proteins based on K19 KRAS binding proteins, as constructed by different E3 ligases, could achieve the degradation of the KRAS protein. Wherein, adoption of E3 ligases βTrCP, SKP2, CRBN, CHIP, SPOP and VHK had achieved better protein degradation effects.

**Table 13:**

| Name of RNA sequence | Percentage of KRAS protein level |
|---|---|
| EV29-βTrCP-K19 | 19.3% |
| EV29-FBW7-K19 | 45.9% |
| EV29-SKP2-K19 | 20.4% |
| EV29-CRBN-K19 | 28.2% |
| EV29-DDB2-K19 | 52.6% |
| EV29-K19-SOCS2 | 47.3% |
| EV29-K19-ASB1 | 39.8% |
| EV29-K19-CHIP | 28.3% |
| EV29-K19-SPOP | 22.4% |
| EV29-K19-VHK | 26.8% |

The aforementioned examples of the present disclosure are merely examples for clear explanation of the present disclosure, and are not intended to limit the embodiments of the present disclosure. For those of ordinary skill in the art, other different forms of changes or variations can be further made on the basis of the above description. All embodiments need not and cannot be exhaustive here. Any modification, equivalent substitution and improvement made within the spirit and principle of the present disclosure shall be included in the protection scope of the claims of the present disclosure.

## Claims

1. A circular RNA molecule, wherein the circular RNA molecule encodes a recombinant polypeptide having a targeting effect activity which is an activity of targeted degradation of a protein of interest.

2. The circular RNA molecule according to claim 1, wherein the recombinant polypeptide comprises a first polypeptide that binds to the protein of interest in a targeted manner and a second polypeptide fused with the first polypeptide, and the second polypeptide has an enzyme activity of a E3 ubiquitin ligase or as an adaptor to mediate E3 ubiquitin ligase activity; and
optionally, the recombinant polypeptide further comprises a linker peptide which links the first polypeptide with the second polypeptide.

3. The circular RNA molecule according to claim 1 or 2, wherein the protein of interest is a transmembrane protein or an intracellular protein; preferably, the protein of interest is a disease-associated protein; and optionally, the disease-associated protein is a tumor-associated protein.

4. The circular RNA molecule according to claim 2 or 3, wherein the first polypeptide is an antibody, antibody fragment or antigen-binding polypeptide that binds to the protein of interest in a targeted manner.

5. The circular RNA molecule according to any one of claims 2-4, wherein the second polypeptide is selected from any domain of a E3 ubiquitin ligase, or a E3 ubiquitin ligase complex; preferably, the second polypeptide is selected from CRBN, VHL, MDM2, cIAP, βTrCP, FBW7, SPOP, SKP2, DDB2, SOCS2, ASB1, CHIP, or a functional fragment of any one of the above.

6. The circular RNA molecule according to any one of claims 1-5, wherein the circular RNA molecule comprises a coding region sequence encoding the recombinant polypeptide and an IRES sequence operably linked to the coding region sequence.

7. The circular RNA molecule according to claim 6, wherein the circular RNA molecule further comprises one or two or more of the following sequences: a second exon sequence, a first exon sequence, a 5' spacer sequence and a 3' spacer sequence; and
preferably, the circular RNA molecule comprises the following sequences connected sequentially: the second exon sequence, the 5' spacer sequence, the IRES sequence, the coding region sequence, the 3' spacer sequence and the first exon sequence.

8. The circular RNA molecule according to any one of claims 1-7, wherein the targeting effect activity is an activity of targeted degradation of a protein of interest in a subject; preferably, the circular RNA molecule is administered into the subject; and
optionally, the circular RNA molecule is administered orally, intraperitoneally, intravenously, intra-arterially, intramuscularly, intradermally, subcutaneously, transdermally, nasally, rectally, by intratumoral injection, by tumor intraluminal indwelling, by intrathecal injection, by subarachnoid cavity injection or systemically, and preferably by intratumoral injection.

9. A cyclization precursor RNA molecule, wherein the cyclization precursor RNA molecule is cyclized to form the circular RNA molecule according to any one of claims 1-8; and
optionally, the cyclization precursor RNA molecule comprises the following sequences connected sequentially:
a 5' homologous arm sequence, a 3' intron sequence, a second exon sequence, a 5' spacer sequence, an IRES sequence, a coding region sequence, a 3' spacer sequence, a first exon sequence, a 5' intron sequence and a 3' homologous arm sequence.

10. A recombinant nucleic acid molecule, wherein the recombinant nucleic acid molecule is transcribed to form the cyclization precursor RNA molecule according to claim 9.

11. A recombinant expression vector, comprising the recombinant nucleic acid molecule according to claim 10.

12. A recombinant host cell, comprising the circular RNA molecule according to any one of claims 1-8, the cyclization precursor RNA molecule according to claim 9, the recombinant nucleic acid molecule according to claim 10, or the recombinant expression vector according to claim 11.

13. A composition, comprising the circular RNA molecule according to any one of claims 1-8; and optionally further comprising one or more pharmaceutically acceptable carriers.

14. Use of the circular RNA molecule according to any one of claims 1-8, the cyclization precursor RNA molecule according to claim 9, the recombinant nucleic acid molecule according to claim 10, the recombinant expression vector according to claim 11, the recombinant host cell according to claim 12 or the composition according to claim 13 in one of the following (a) or (b):
(a) targeted degradation of a protein of interest;
(b) reducing or eliminating the expression of a protein of interest.

15. The circular RNA molecule according to any one of claims 1-8, the cyclization precursor RNA molecule according to claim 9, the recombinant nucleic acid molecule according to claim 10, the recombinant expression vector according to claim 11, the recombinant host cell according to claim 12 or the composition according to claim 13 for use in preventing or treating a disease in a subject, wherein the disease is prevented or treated with the circular RNA molecule, the cyclization precursor RNA molecule , the recombinant nucleic acid molecule, the recombinant expression vector, the recombinant host cell or the composition via administration ;
wherein optionally, the administration is selected from oral administration, intraperitoneal administration, intravenous administration, intra-arterial administration, intramuscular administration, intradermal administration, subcutaneous administration, transdermal administration, nasal administration, rectal administration, intratumoral injection, tumor intraluminal indwelling, intrathecal injection, subarachnoid cavity injection or systemic administration, preferably intratumoral injection;
preferably, the disease is cancer; more preferably, the disease is selected from lung cancer, liver cancer, stomach cancer, colon cancer, and metastatic forms thereof.
